# EUROPEAN PATENT SPECIFICATION

(11) **EP 3 052 166 B1**
(45) Date of publication and mention of the grant of the patent: **17.04.2019**
(21) Application number: 14789667.4
(22) Date of filing: 30.09.2014
(51) Int. Cl.: A61M 5/20, A61M 5/24, A61M 5/315, A61M 5/32

(54) **ADHESIVE COVER PEELER AND NEEDLE COVER REMOVER FOR AUTOINJECTOR**
KLEBER SCHUTZFOLIE SCHÄLER UND
NADELABDECKUNG ENTFERNER FÜR AUTOINJEKTOR
COLLE PROTECTION EPLUCHEUR ET
EXTRACTEUR D'AIGUILLES DE COUVERTURE POUR AUTO-INJECTEUR

(30) Priority: 30.09.2013 US 201361884597 P; 04.12.2013 US 201314096977; 21.02.2014 US 201414186403; 30.06.2014 US 201462019066 P
(43) Date of publication of application: 10.08.2016
(73) Proprietor: Medimop Medical Projects Ltd., 43665 Ra'anana (IL)
(72) Inventor: CABIRI, Oz, 71799 Macabim-Reut (IL)
(74) Representative: Lippert Stachow Patentanwälte Rechtsanwälte
(86) International application number: PCT/US2014/058433
(87) International publication number: WO 2015/048791

(56) References cited:
- WO-A1-2012/134588
- US-A- 4 781 688
- US-A1- 2002 022 798

## Description

### BACKGROUND OF THE INVENTION

The present invention, in some embodiments thereof, relates to a composite medical according to the generic part of claim 1 and, more particularly, but not exclusively, to synchronized preparing for use of internal and external components of a composite medical device.

U.S. Patent Application Publication No. 2011/0098656 to Burnell discloses an injection device having a housing that receives a syringe having a sealed boot that covers its needle. The syringe is biased by a return spring from an extended position in which the needle extends from the housing through an exit aperture to a retracted position in which it does not. A drive spring acts via a drive to advance the syringe from its retracted position to its extended position and discharge its contents through the needle and a return the spring, brought into play when the drive has reached a nominal return position, restores the syringe to its retracted position. A releasable locking mechanism retains the syringe in its retracted position. A sleeve projects from the exit aperture and can be depressed to release the locking mechanism. A removable threaded cap closes the housing, covers the exit aperture and the sleeve, thus preventing the locking mechanism from being released, and engages the boot an the syringe. When the cap is removed, it takes the boot with it, no longer closes the exit aperture and no longer prevents the locking mechanism from being released. Then, the locking mechanism can be released and the injection cycle begun.
U.S. Patent Application Publication No. 2013/0310753 to Cabiri discloses a method for selectively powering a battery-operated drug-delivery device, the device having a battery and a battery circuit, the method comprising: providing a battery isolator in a first position whereat it interrupts a battery circuit, whereby no power is provided to the device; and activating a fastening mechanism configured for fastening the device to a user, the activating causing the battery circuit
to be uninterrupted by the isolator, such that power is provided to the device. Additionally, there is provided a selectively powered battery-operated drug-delivery device, comprising: a selectively-removable isolator disposed in a first position whereat it interrupts a battery circuit; the isolator movable to a second position whereas the battery circuit is uninterrupted by the isolator; and a mechanism for fastening the device to a user, activation of the fastening mechanism moving the isolator from the first position to the second position.

U.S. Patent Application Publication No. 2012/0323183 to Peterson discloses a drug delivery device, including a body having a needle opening and a reservoir disposed therein for containing a medicament, and an injection needle for penetrating the skin of a patient, the needle providing a path for the medicament between the reservoir and the patient, and selectively protruding from the body through the needle opening. The device also includes safety means for automatically retracting the needle within the body and covering the needle opening upon removal of the device from the patient.

U.S. Patent Application Publication No. 2005/0049561 to Hommann discloses a device for administering an injectable product including a casing, an injection mechanism including an injection needle pointing in an insertion direction, and a protective cap for the injection needle wherein, in one embodiment, the administering device includes a removing device for removing the protective cap from the injection needle and, in another embodiment, the administering device includes a needle protecting sleeve shiftable generally in alignment with the casing and generally between a front position, advanced relative to the casing, for protecting the injection needle and
to a rear position, retracted relative to the casing, for inserting the injection needle into a tissue. In one embodiment, the needle protecting sleeve is prevented from moving completely into the rear position by a lock when the protective cap is protecting the injection needle.

U.S. Patent Application Publication No. 2003/0229308 to Tsals discloses an injector adapter that permits an automatic injector to be releasably coupled to the skin of a living being
without the need for a user to hold the injector against the skin during injection, especially in cases where the drug being delivered requires a slow rate of injection. In addition, another variation of the invention is described whereby the working end of the automatic injector includes an integrated injector adapter.

US 4,781,688 discloses a dispensing device having an interior volume and an exterior surface comprising a wall located between the interior volume and an exterior of the compound device, a protected component surrounded by an enclosure, said enclosure located in the interior volume, a seal for closing said enclosure and for isolating said protected component from said wall, an active area formed an at least a portion of the exterior surface, a cover shielding said active area from said exterior, and a coupler attaching said seal to said cover, said coupler synchronizing removal of said cover and unsealing of said enclosure.

Additional background art includes International Patent Application Publication No. WO 2001052925 to Sterling Medivations Inc, U.S. Patent 7530964 to Cabiri, U.S. Patent No. 8,348,898 to Cabiri, U.S. Patent Application Publication No. 2009/093,792 to Gross and Cabiri, International Patent Application Publication No. WO/2013/104414 to SANOFI-AVENTIS DEUTSCHLAND GMBH, U.S. Patent Application Publication No. 2011/0166509 to Gross and Cabiri, U.S. Patent Application Publication No. 2012/0130344 to Ebbett, U.S. Patent No. 8,267,890 to Alchas and U.S. Patent Application Publication No. 2009/0012494 to Yeshurun.

### SUMMARY OF THE INVENTION

In accordance with an aspect of some embodiments of the present invention there is provided a compound device according to claim 1. The compound device is provided with an interior volume and an exterior surface including: a wall located between the interior volume and an exterior of the compound device; a protected component surrounded by an enclosure, the enclosure at least partially located in the interior volume; an opening in said wall exposing said enclosure to said exterior; an active area formed on at least a portion of the exterior surface; a cover (1085) shielding said active area (578) from said exterior. According to the present invention a seal for closing the enclosure and for isolating the protected component from the wall is provided; an active area is formed on at least a portion of the exterior surface; a coupler is provided attaching said seal to said cover, wherein said coupler connects said seal to said cover via said opening and wherein said coupler is adapted to synchronize removal of said cover and unsealing of said enclosure. In accordance with some embodiments of the invention, the compound device further includes an opening in the wall exposing the enclosure to the exterior.

In accordance with some embodiments of the invention, the active area is adjacent to the opening.

In accordance with some embodiments of the invention, the coupler connects the seal to the cover via the opening.

In accordance with some embodiments of the invention, the active area includes an adhesive.

In accordance with some embodiments of the invention, the protected component includes a hypodermic needle.

In accordance with some embodiments of the invention, the seal includes a needle protective sleeve.

In accordance with some embodiments of the invention, the compound device further includes an opening in the wall exposing the enclosure to the exterior and the hypodermic needle is directed to protrude through the opening in the wall.

In accordance with some embodiments of the invention, the coupler converts a movement away from the exterior surface into a peeling forte on the cover.

In accordance with some embodiments of the invention, the coupler is connected to an edge of the cover.

In accordance with some embodiments of the invention, the coupler is flexible.

In accordance with some embodiments of the invention, the coupler includes a slack allowing movement of said seal at least 4mm before commencing said peeling.

In accordance with some embodiments of the invention, a volume of the enclosure is smaller than a volume of the interior volume.
In accordance with an aspect of some embodiments of the present invention there is provided a method of preparing a device for use, the device including an active outer surface and a protected component isolated from a wall of the device by a sealed enclosure; the active outer surface protected by a surface cover, the method including: activating the active outer surface by removing the surface cover; exposing the protected component to the wall of the device by unsealing the sealed enclosure and synchronizing the activating and the unsealing using a coupler attached to the surface cover and the sealed enclosure.

In accordance with some embodiments of the invention, the activating includes peeling the surface cover from the active outer surface.

In accordance with some embodiments of the invention, the method further includes pulling the coupler away from the surface cover to form a space prior to the peeling.

In accordance with some embodiments of the invention, the protected component includes a hypodermic needle, and the peeling force is applied on an edge of the cover.

In accordance with some embodiments of the invention, the unsealing includes pulling a needle protective sleeve along an axis of a needle.

In accordance with some embodiments of the invention, the protected component includes a hypodermic needle, the method further including: projecting the hypodermic needle through an opening in the wall.

In accordance with some embodiments of the invention, the active surface is adjacent to the opening.

In accordance with an aspect of some embodiments of the present invention there is provided a method of removing a cap and peeling a cover from a surface including: providing a coupler joining the cap to the cover; pulling the cap and a portion of the coupler joined thereto away from the surface and converting of the pulling force on the coupler to a peeling force on the cover of the surface.

In accordance with some embodiments of the invention, the method further includes applying the peeling force on an adhered edge of the cover.

In accordance with some embodiments of the invention, the cap includes a needle protective sleeve and the pulling is along an axis of a needle.

In accordance with some embodiments of the invention, the cap protects a sterility of a hypodermic needle and the method further including: projecting the hypodermic needle through an opening in the surface.

In accordance with an aspect of some embodiments of the present invention there is provided a method of preparing a device for use including: supplying a component of the device sealed by a seal; protecting an external surface of the device with a cover; joining the seal to the cover such that breaking seal and removing the cover are synchronized. Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:
FIG. 1A is a flowchart illustrating a method of enabling a compound device in accordance with an embodiment of the present invention;
FIG. 1B is a schematic block diagram of a compound device in accordance with an embodiment of the present invention;
FIG. 2 is a flowchart illustrating a method of enabling a compound device in accordance with an embodiment of the present invention;
FIG. 3 is a state diagram of an injector in accordance with an embodiment of the present invention;
FIG. 4 is a flowchart illustrating a method of manufacture of a compound device in accordance with an embodiment of the present invention;
FIG. 5A is an exploded view illustrations of a stabilized injector in accordance with an embodiment of the present invention;
FIG. 5B is an orthogonal silhouette view of a stabilized injector in accordance with an embodiment of the present invention;
FIG. 5C-D are schematic perspective view illustrations of a surface cover for shielding an active surface of a compound device in accordance with an embodiment of the present invention;
FIG. 5E-H are a cross sectional illustrations of peeling a surface cover in accordance with an embodiment of the present invention;
FIG. 5I is an exploded view illustrations of a surface cover in accordance with an embodiment of the present invention;
FIG. 5J is an orthogonal view illustrations of a surface covcr in accordance with an embodiment of the present invention;
FIG. 6A is a cross sectional illustration of an alternative enabling assembly for a compound device in accordance with an embodiment of the present invention;
FIG. 6B-C are schematic illustrations of an alternative surface cover for shielding an active surface of a compound device in accordance with an embodiment of the present invention;
FIG. 7A-C are a cross sectional illustrations of peeling an alternative surface cover in accordance with an embodiment of the present invention;
FIG. 8 is a cross sectional illustration of peeling an alternative surface cover in accordance with an embodiment of the present invention;
FIG. 9 is a cross sectional illustration of peeling an alternative surface cover in accordance with an embodiment of the present invention;
Fig. 10A illustrates an external perspective view of a patch injector in accordance with an embodiment of the current invention;
Fig. 10B illustrates a perspective view of the internal parts of a patch injector in accordance with an embodiment of the current invention;
Fig. 10C illustrates an external perspective view of a patch injector including a surface cover and coupler in accordance with an embodiment of the current invention;
Fig. 10D illustrates a cross sectional view of enabling assembly of a patch injector in accordance with an embodiment of the current invention;
Figs. 11A-B illustrate a cross sectional views an alternative enabling assembly of a patch injector in accordance with an embodiment of the current invention;
Fig. 12A illustrates an external perspective view of an alternative patch injector in accordance with an embodiment of the current invention;
Fig. 12B illustrates a perspective view of the internal parts of an alternative patch injector in accordance with an embodiment of the current invention;
Fig. 12C illustrates an external perspective view of an alternative patch injector including a surface cover and coupler in accordance with an embodiment of the current invention;
Figs 13A-C illustrate a needle cover and an injector safety cover including a needle cover remover in accordance with an embodiment of the present invention;
FIGS. 14A-C illustrate an injector with safety cover and adhesive cover in accordance with an exemplary embodiment of the present invention;
FIG. 15 is a flow chart illustration of a method of manufacture of a stabilized pen injector in accordance with an embodiment of the present invention;
FIG. 16 is a flowchart illustration of a method of stabilizing an autoinjector in accordance with some embodiments of the current invention;
FIG. 17 is a block diagram of an autoinjector and a stabilizing adaptor in accordance with some embodiments of the current invention; and
FIG. 18 is a state diagram of an autoinjector and extending adaptor in accordance with some embodiments of the current invention.

### DETAILED DESCRIPTION OF THE INVENTION

The present invention, in some embodiments thereof, relates to a composite medical and, more particularly, but not exclusively, to preparing a composite medical device for use.

### Overview

### 1 Activating a surface and unsealing an isolated zone

An aspect of some embodiments of the present invention relates to enabling a compound device requiring peeling a cover for example from an active surface and exposing a protected component isolated from the surface. The peeling and unsealing may optionally be synchronized. For example, the surface may include a non-sterile or low-level sterile adhesive to be adhered to the skin of a patient and the protected component may include a highly sterile implement for insertion into tissue of the patient. For example, the protected component may be in an isolated enclosure located behind an opening in the adhesive surface. An adhesive cover may be joined through the opening to a seal of the isolated enclosure. Optionally, peeling away the adhesive cover may unseal the enclosure exposing the protected component to the opening. In some embodiments the adhesive surface may be sterile. Optionally the adhesive cover may protect the sterility of the adhesive.

### 2 Coupler to peel a surface cover with an axial force

An aspect of some embodiments of the present invention relates to an apparatus that converts a movement away from a surface to peeling movement along the surface. For example a device may optionally include a cap that is removed by pulling linearly away from the body of the device. The device may optionally include a surface cover that may be removed from a surface by peeling. In some embodiments, a coupler may connect between the cap and the surface cover. Optionally, while the cap is being pulled off, the coupler may convert the pulling force on the cap to a peeling force on the surface cover. In some embodiments, the coupler may apply a force that tears a sealed packaging. For example, the sealed packaging may protect a sterility of the adhesive. Optionally the peeling force may be applied to an edge of the surface cover. For example the coupler may be flexible and/or a hinged and/or may be anchored to an edge of the surface cover. In some embodiments, the coupler may include slack that allows limited movement of the cap before the commencement of peeling. For example, the slack may allow movement of the cap to a certain distance away from the body for example to get an improved leverage on the peeling before peeling begins. For example the slack may allow movement of the cap and/or handle and/or a seal a distance ranging between 1mm and 4 mm and/or a distance between 4mm and 10mm and/or between 10mm and 20mm and/or more than 20mm before starting peeling of the surface cover.

### 3 Application to a medical injector

An aspect of some embodiments of the present invention relates to a mechanism to synchronize peeling an adhesive cover and exposing needle an autoinjector. For example the safety cap may include a needle protector remover. Optionally, safety cap and/or the needle protector remover may be attached to an adhesive cover. The safety cover may optionally protect the device from uncontrolled early activation. Removing the safety cap optionally includes removing a sterile needle cover. As the safety cap is pulled away the pulling force may be transferred to a peeling force at one or more edges of the adhesive cover. In some embodiments the autoinjector may be an integrated system. Alternatively or additionally the autoinjector may include a non-stabilized injector system (for example a conventional pen injector). Optionally, the adhesive and/or the adhesive cover and/or the safety cover and/or the coupler may be included in an adaptor attached to the injection system. Alternatively or additionally the autoinjector may include a non-stabilized injector system (for example a conventional pen injector) including a needle protection cover and/or a safety cover. Optionally the adhesive and/or the adhesive cover and/or the coupler may be included in an adaptor attached to the injection system.

Before explaining at least one embodiment of the invention in detail, it is to be understood that the invention is not necessarily limited in its application to the details of construction and the arrangement of the components and/or methods set forth in the following description and/or illustrated in the drawings and/or the Examples. The invention is capable of other embodiments or of being practiced or carried out in various ways.

### Exemplary embodiments

### 1 Unsealing a protected component and activating a surface

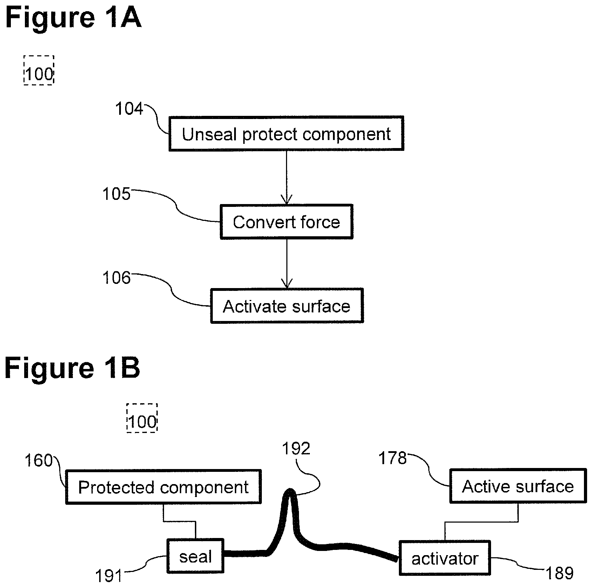

Referring now to the drawings, Fig. 1a illustrates a method of synchronizing unsealing of a protected component and peeling of a surface cover in accordance with an embodiment **100** of the present invention. For example a protected component may be unsealed **104** (for example by removing and/or breaking and/or melting a seal [for example seal **191** of Fig. lb]). For example, the unsealing may be by means of application of a linear and/or twisting force to the sealed component. The unsealing force may be converted **105** to an activating **106** energy on an active surface of the device.

Referring now to the drawings, Fig. 1b is a block diagram illustration of a system for synchronizing unsealing **104** of a protected component and activation **106** of a surface in accordance with an embodiment **100** of the present invention. For example, some embodiments may include a seal **191** for isolating one or more protected components **160** and/or an activator **189** for an active surface **178.** Optionally seal **191** and activator **189** may be joined by a coupler **192.** Coupler **192** may convert **105** an energy unsealing **104** seal **191** to activate **106** of an active surface. In some embodiments coupler **192** may include slack, for example a fold and/or wrinkle and/or an elasticity which allows some individual movement of seal **191** and/or activator **189** before synchronized movement begins.

### 2 Method of injecting a drug

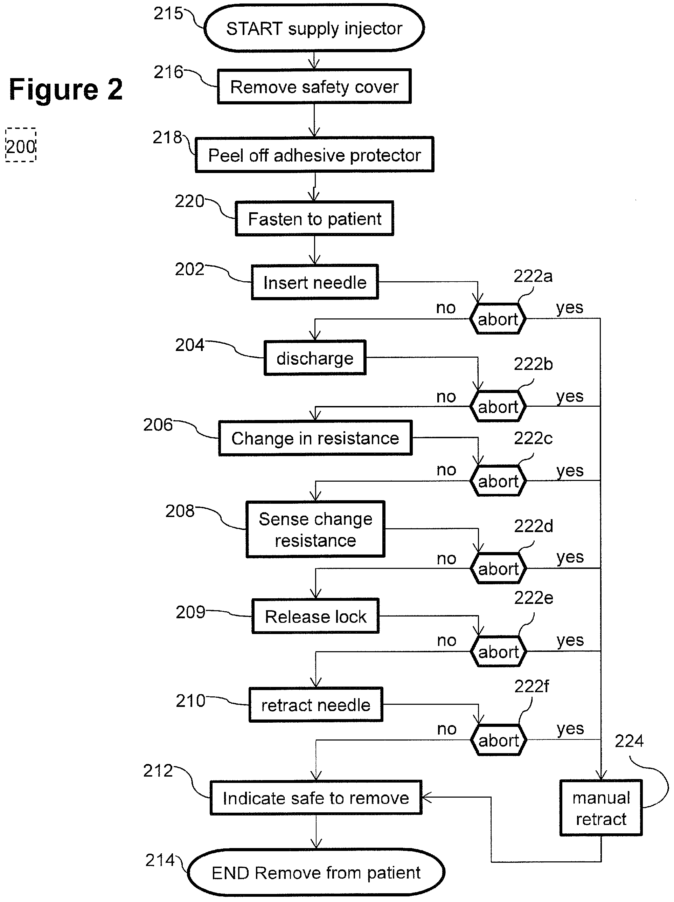

Referring now to the drawings, fig. 2 illustrates a method of injecting a drug in accordance with an embodiment **200** of the present invention. In the exemplary method, a needle is optionally held stable while a drug is dispensed to a patient (dispensing a drug may include discharging a drug from the injector).

In exemplary embodiment **200** a user (for example a patient and/or a medical aid in home care) may be supplied **215** with an autoinjector ready to administer a medicine.

The user may optionally remove **216** a safety cover from the injector. Removing **216** the cover may optionally include removing a sterile cover from a needle. Removing **216** the cover may optionally automatically peel **218** an adhesive protector from an adhesive. For example, the adhesive may be supplied to stabilize the injector on the skin of a patient during injection. The injector may optionally be fastened **220** to a patient (who may be the user). In some embodiments a user may hold the injector to the skin of a patient; the fastening **220** may stabilize the injector for example from shifts and/or movements of the patient and/or the user.

In some embodiments, the user may set off an activation mechanism. The activation mechanism may for example insert **202** the needle into the patient, for example by extending the needle outward. For example, a syringe may be moveably attached to the base. A syringe may optionally be rigidly attached to the syringe. For example the syringe may slide linearly along its axis. Sliding the syringe towards the base may cause the needle rigidly to protrude beyond the base. For example, part of the needle may pass through a hole in the base and pierce the skin of a patient. The adhesive of the base may hold the skin of the patient steady while the needle pierces the skin. The combination of an adhesive holding the skin and moving the needle to a predetermined position past the base may facilitate the inserting **202** of the needle into the skin to the desired depth.

The needle may optionally be locked in the extended position. Optionally, the needle may be biased to a protected position (for example to retract into a housing of the injector). Alternatively or additionally, the needle may be biased to the unshielded position. Alternatively or additionally, the autoinjector may be supplied with the needle in an extended mode and/or protected by a cover.

At a point during the injection process, an optional manual retraction **224** mechanism may be used to place the injector in a safeguarded mode. For example, when the user decides to abort **222a-f** at a point in the process (for example when he detects some sort of malfunction and/or feels a negative reaction to the medicine) the user may manually retract **224** the needle. Optionally there may be an indicator to indicate **212** whether the needle was automatically retracted **210** and/or whether needle was manually retracted **224.** Alternatively or additionally there may be an indicator whether a full dose was administered and/or how much medicine was administered.

Once the needle is inserted into the patient, the injector may optionally begin discharging **204** medicine. For example the medicine may be injected through the needle into the patient. Optionally, discharge may continue until a full dose of the medicine is administered.

In some embodiments, after administration of a full dose of the medicine, there may be a change **206** in resistance to further discharging. For example in a syringe based injector, a plunger may reach the end of the syringe and cease to move increasing resistance. Alternatively or additionally, after discharging the entire dose a transmission may be disconnected (for example a threaded element may pass the end of its threading) reducing resistance. Alternatively or additionally, the change **206** in resistance may result from another cause for example increased resistance due to a full or partial occlusion of a fluid pathway and/or jamming of a mechanical component (for example cross threading of a screw). The change of resistance may optionally be sensed **208** triggering retracting **210** of the needle.

In some embodiments, the needle may be locked in an unshielded state by a force sensitive lock. When the lock senses **208** the change **206** in resistance, it may release **209** the needle which may be retracted **210** to a shielded position.

In some embodiments, a flag may be supplied (for example a LED and/or a changing color indicator) to indicate **212** to the user that the needle has been retracted **210** and/or that the injector can safely be removed **214** from the patient and/or that a fastener has been released. For example, if the injector is adhered to the patient, it may be peeled off and/or a fastener may be released.

### 3 States of an autoinjector

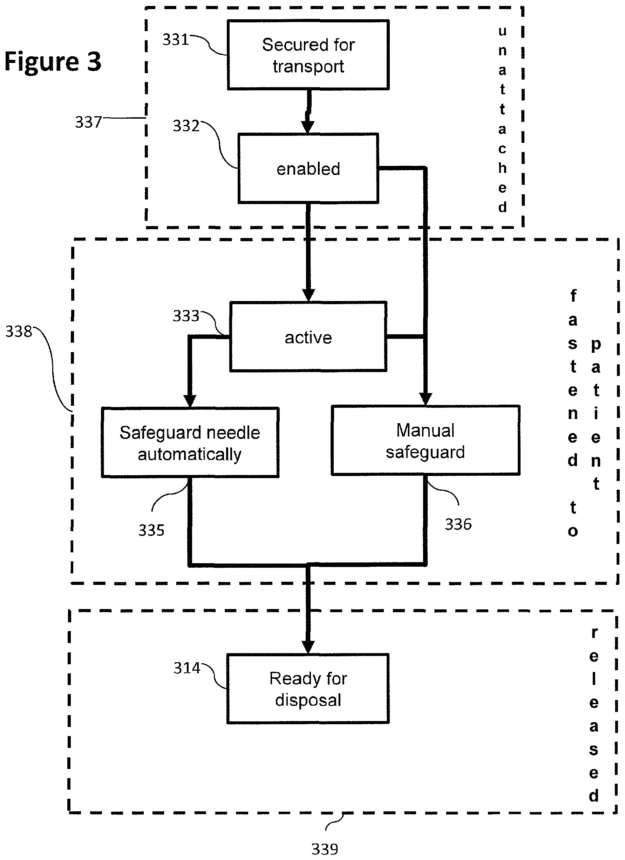

Fig. 3 is a state diagram of an autoinjector in accordance with an embodiment of the present invention. In general, may be supplied in an unattached **337** state. An unattached **337** autoinjector may have a secured **331** state. For example in the secured **331** state the injector may be safe to handle and/or transport. Optionally the injector may have an enabled **332** state. For example, in the enabled **332** state, the injector may be unstable and/or easily activated. For example, an injector may be switched from the secured **331** state to the enabled **332** state by removing a needle protector and/or an adhesive cover.

Once activated the injector may optionally be fastened to a patient. In the fastened **338** state the injected may optionally be activated. For example, while the injector is in the active **333** state, a needle may project from the injector. In some embodiments the injector may be hazardous to handle in the enabled **332** and/or active **333** state.

In some embodiments, after use (optionally whether or not administration of the full dose was successful) the user may want to remove and/or dispose of the autoinjector. In some embodiments, it may be difficult and/or dangerous to remove an injector in the enabled and/or active state. For example, when an injector is fastened to a patient by an adhesive, it may be difficult to remove the needle by pulling the injector away from the skin. Optionally, first a needle may be retracted from the skin into the injector. Subsequently the adhesive may be removal by peeling from the skin. In some embodiments, the injector may automatically be safeguarded **335** for example by retraction of a needle upon completion of injection. Alternatively or additionally, the user may have the option to manually secure the injector into a safeguarded **336** state. For example, the optionally of manually needle retraction may avoid the situation where a patient may not be able to properly remove the injector due to a malfunction that leaves the injector fastened to the skin with the needle inserted into the patient. During and/or after safeguarding **335, 336** the injector may be removed from the patient.

Optionally, the injector may have a final released state **339,** for example wherein the needle is retracted back into the injector and/or the needle tip is shielded and/or the injector has been unfastened from the patient. Optionally one or more indicators may be supplied to indicate the state of the injector and/or the quantity of medicine discharged. Once released, the injector may be in final **314** state (protected from hazards and/or ready for disposal, for example in a municipal waste).

### 4 Method of manufacture of an compound device

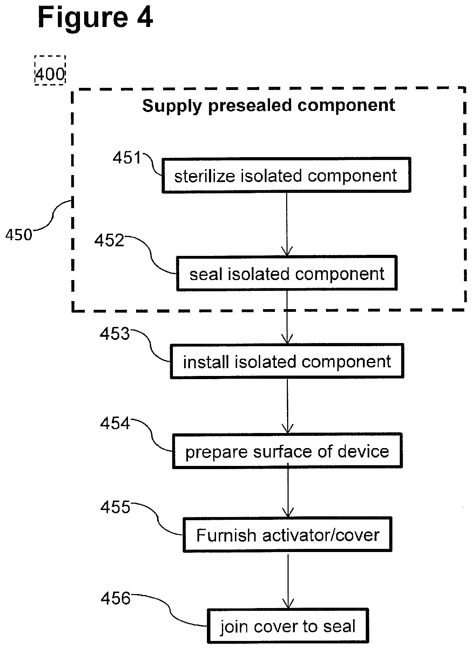

Fig. 4 is a flow chart illustration of a method of manufacture of a compound device in accordance with an embodiment of the present invention. In some embodiments, a protected component may be installed into a device in a sealed state. The device may optionally include an active surface with an activation mechanism. Optionally, the surface activator may be joined to a seal of the protected component, for example by a coupler. Optionally, the coupler may synchronize unsealing of the protected component and activation of the active surface. For example the method of manufacture illustrated in embodiment **400** of Fig. 4 may optionally be used in manufacturing one, some and/or any of the embodiments of an injector illustrated in the embodiments illustrated herein above and/or below. In some embodiments, a sterile adhesive may be sealed in a protective packaging. The adhesive may for example be attached to a surface in a sterile and/or protected state. Optionally the coupler may be connected to the protective packaging of the adhesive and/or may synchronize opening of the packaging with another act.

In some embodiments an isolated component may be supplied **450** in a presealed state. For example, the presealed component may be a fluid path of a preloaded syringe. Optionally the syringe and/or fluid path may be sterilized **451** and/or preloaded with for example medicine and/or sealed **452** in an aseptic room.

In some embodiments, a protected component may be installed **453** into a device in a sealed state. For example, in some embodiments, a preloaded syringe with a sealed sterile fluid path may optionally be installed **453** into an autoinjector.

In some embodiments, the device may optionally include an active surface with an activation mechanism. The active surface may be prepared **454.** For example an autoinjector may include an active surface including an adhesive for attaching to a patient. Preparing **454** the surface may include for example applying the adhesive to the surface.

In some embodiments, the active surface may be furnished **455** with an activation mechanism and/or protective cover. For example, in some embodiments, an adhesive surface may be covered by an adhesive cover and/or activated by removing an adhesive cover.

In some embodiments, the surface cover and/or activator may be joined **456** to the seal of the protected component, for example by a coupler. Optionally, the coupler may synchronize unsealing of the protected component and activation of the active surface. For example removing the surface cover and/or activating the surface may trigger unsealing of the protected component. Alternatively or additionally, unsealing of the protected component may trigger removal of the surface cover and/or activating of the active surface.

### 5 Stabilized injector with Double folded surface cover

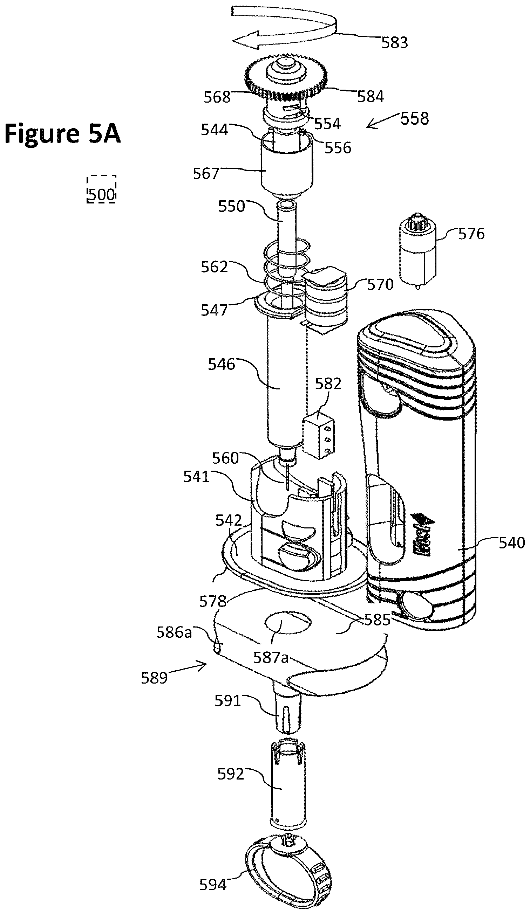

Figs. 5A-K illustrate an exemplary embodiment of a compound device including an isolated component protected by a seal and an active surface activated by removing a cover and a coupler joining the seal and the cover for synchronized removal in accordance with some embodiments of the current invention. Optionally, in the exemplary embodiment the surface cover is doubly folded, forming dual extensions that convert a linear unsealing force into a balanced peeling force on opposite edges of adhesion of the surface cover.

Fig. 5A illustrates an exploded view of a stabilized pen injector in accordance with some embodiments of the current invention. In some embodiments, a prefilled syringe **546** may be installed into the injector. Prior to installation, the syringe may optionally be attached to a sterile fluid path (for example a needle **560**). The sterility of fluid path may be protected from contamination by a seal, (for example needle **560** may be isolated from contamination by being enclosed in a needle cover **591** [which is illustrated for example as a needle protective sleeve]). Syringe **546** and/or needle **560** may optionally be installed into injector **500** is a sterile sealed state.

Injector **500** may include for example an adhesive **578** base **542.** In some embodiments, an adhesive **578** surface formed on a wall (for example a base **542** of injector **500**) may assist a user to hold injector **500** steady on the skin of a patient for an extended period. For example, injector **500** may be used to give injections of volume ranging between 0.5 and 3.0 ml over a time period ranging between 30 sec to 180 sec.

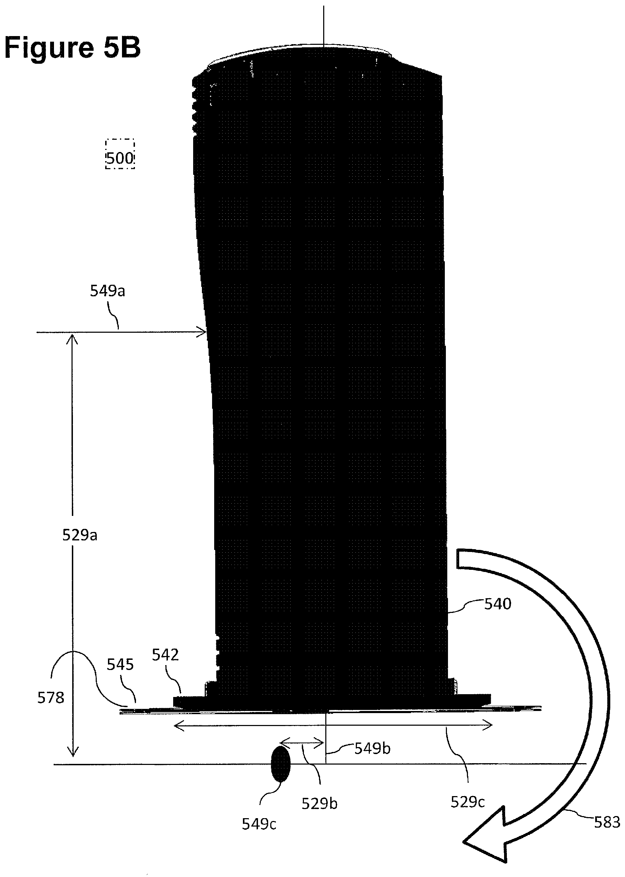

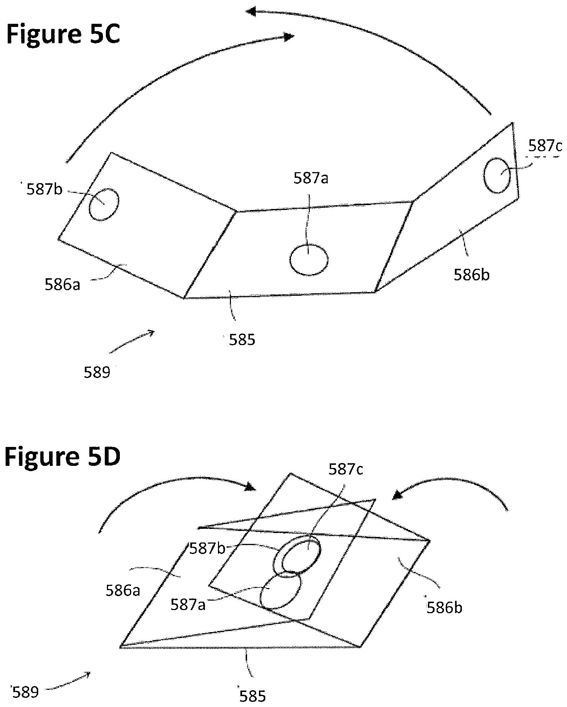

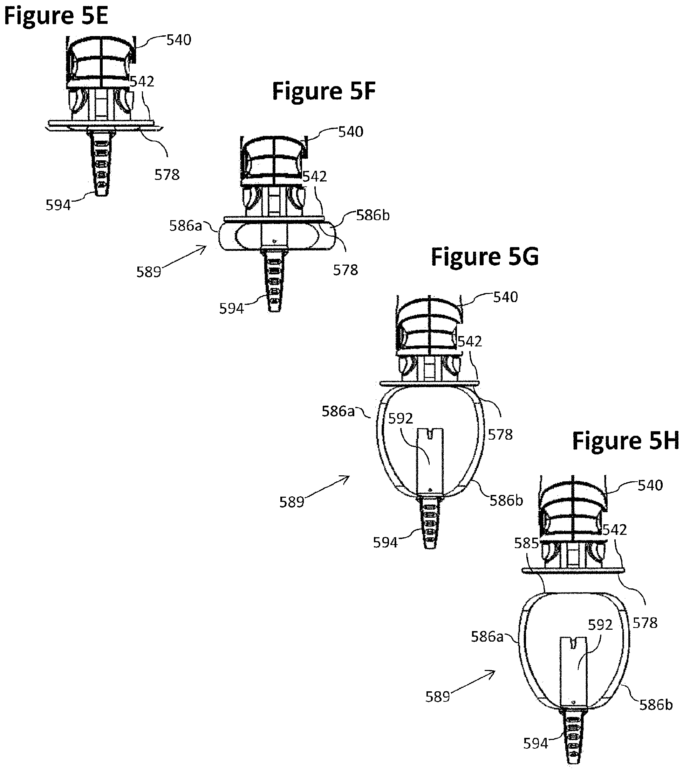

In some embodiments, a coupler (for example safety cap **592**) may join needle cover **591** to adhesive cover **589.** For example, safety cap **592** may include extenders **586a,b** (for example see Fig. 5B) for attaching to adhesive cover **589.** Safety cap **592** may optionally have the form of a sleeve and/or a clamp that is inserted through an opening in adhesive cover **589** (for example opening **587a** in the adhered portion **585** and/or openings **587b,c** in extenders **586a,b** for example see Fig. 5C) and/or an opening **587d** (for example see Fig. 5E) in base **542.** Optionally, safety cap is inserted into injector housing **540** and/or is attached to needle cover **591** after assembly of the injector. Extenders **586a,b** may optionally be attached to and/or be an integral part of safety cap **592.** Optionally a handle **594** may clamp extenders **586a,b** to safety cap **592.** For example, a coupler for a handle **594** may fit through holes **587b,c** in the extenders **586a,b** clamping them to safety cap **592.**

Optionally injector **500** may be loaded with a standard type syringe **546** and/or hypodermic needle **560.** For example, needle **560** may be rigidly connected and/or project from a distal end of syringe **546.** Needle **560** may be coaxial with syringe **546.** Alternatively or additionally the axis of needle **560** may be parallel to the primary longitudinal axis of syringe **546** but offset therefrom. In some embodiments, injector **500** may include a shield **541** which extends the distal end of a housing **540** of the injector. In some embodiments, a motor switch **582** may be located in shield **541.** In the enabled state (before activation), switch **582** is optionally switched off.

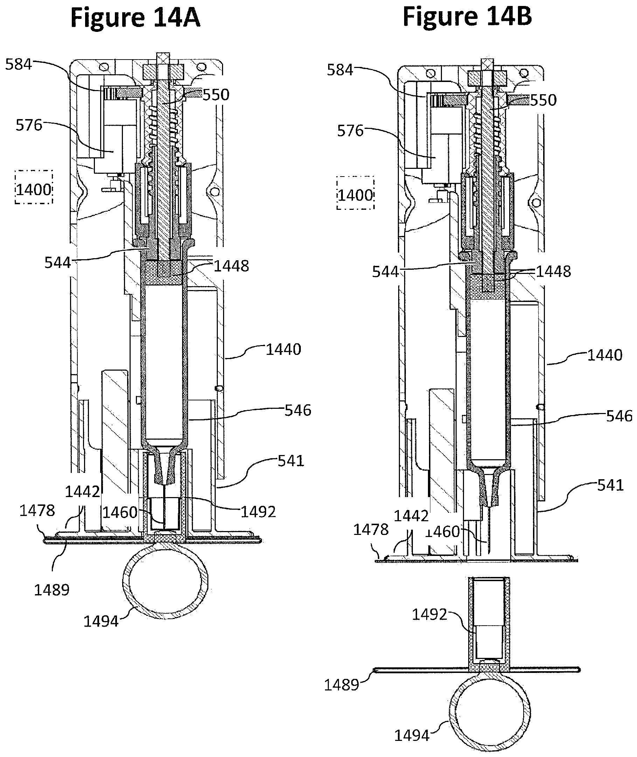

In the exemplary embodiment of injector **500** a power supply (for example batteries **570**) may optionally supply power to gear motor **576.** The motor may optionally turn a threaded assembly and/or a telescoping assembly. For example the threaded assembly may include a rod **550** pushing a plunger [for example see plunger **1448** Fig. 14A-C]). Optionally, syringe **546** may include a flange. For example, flange **547** may have a non-rounded edge which may be held inside injector **500** preventing rotation of syringe **546.**

In some embodiments, an injector may include a retraction mechanism. For example, in injector **500,** a retraction mechanism **558** is optionally activated by a combination of torque and linear stress. For example the combined torque and linear stress may occur when a plunger (for example plunger **1448,** see Fig 14A-C) is blocked. For example, blockage may occur when the plunger reaches the end of injection (and/or for example due to an occlusion of needle **560**).

In some embodiments, during drug discharge a motor (for example motor **576**) rotates transmission **584** in the direction of arrow **583.** Transmission **584** may optionally be rigidly connected to and/or integrally molded with an inner sleeve **568.** Rotating transmission **584** may also rotate inner sleeve **568.** A pin **556** optionally protrudes from a driver **544** into a nearly horizontal portion of a slot **554** in sleeve **568.** In some embodiments, while pin **556** is in the horizontal portion of slot **554,** driver **544** is prevented from moving longitudinally with respect to inner sleeve **568.** In some embodiments syringe **546** is supported (from moving proximally) by driver **544.**

In some embodiments, when there is a strong linear force on driver **544** in the proximal direction and/or there is a strong torque on sleeve **568** in the direction of arrow **583,** pin **556** slides out of the horizontal portion of slot **554** into a longitudinal portion of slot **554.** In longitudinal portion of slot **554** pin **556** may slide longitudinally (in the proximal direction). A geometry of pin **556** and/or an interference element may optionally be chosen to achieve a desired resistance to movement. For example, pin **556** and/or the interference element may have a squared side, a flat side, a rounded side etc.

In some embodiments, a spring (for example spring **562**) biases syringe **546** in the proximal direction. For example spring **562** may apply a proximal force to flange **547.** Optionally another biasing element may be used in place of spring **562.** For example, a biasing element may include a stretched element (for example a rubber band and/or a twisted elements and/or a deflected plastic element). Alternatively or additionally, syringe **546** may be moved up and down by a motor and/or a pulley and/or a screw and/or a hydraulic element and/or the like.

Optionally when pin **556** enters the longitudinal portion of slot **554,** spring **562** may optionally push syringe **546** and/or outer sleeve **567** and/or needle **560** and/or driver **544** and/or pin **556** proximally, retracting needle **560.** Optionally, needle **560** may be held in the retracted position by spring **562.** Alternatively or additionally a locking mechanism may be included to lock needle **560** in the retracted position, for example, a one way catch and/or an interference element may lock against syringe **546** as it is retracted and/or against pin **556** in slot **554.** Optionally, in injector **500** driver **544** includes two molded plastic telescoping pieces. One piece is optionally integrally molded with outer sleeve **567.** Optionally, sleeve **567** and/or driver **544** may be made as a single piece and/or multiple parts. They may be formed of plastic and/or another material and/or they may be molded and/or formed by another process.

Fig. 5B illustrates peeling adhesive cover **589** from an adhesive **578 in** accordance with an embodiment of the present invention. In the embodiment of injector **500** safety cap **592** may optionally serve as a peeler for an adhesive cover **589.** For example, safety cap **592** is attached to extenders **586a,b.** Extenders **586a,b** may optionally connect safety cap **592** to an edge of adhered portion **585** of adhesive cover **589.** For example, in the embodiment of injector **500,** extenders **586a,b** may be formed from adhesive cover **589.** Alternatively or additionally, extenders may be hinged and/or flexible extensions to safety cap **592.** Optionally, pulling handle **594** which may optionally be located at the center of safety cap **592** does not pull adhesive cover **589** directly away from the whole surface of the adhesive **578** all at once (an act that might require a large force to overcome the sticking force over a large surface). When handle **594** at is pulled, safety cap **592** may optionally move away from adhesive cover **589.** Extenders **586a,b** may unfurl, unfold, stretch and/or bend to allow a certain distance to build up. Then they may optionally convert the linear force away from base **542** to a peeling force at the edge of adhesion of adhesive cover adhesive cover. The peeling force may optionally be along the surface of base **542** and/or the peeling force may be directed away from the surface at an angle. For example the example of angle of the peeling may range for example between 60-90 degrees and/or between 30-60 degrees and/or between 0 and 30 degrees. For example extenders **586a,b** may optionally unfold and/or flex in such a way as to peel adhesive cover **589** bit by bit. Peeling may optionally be from an edge of adhesive **578** and/or towards the center.

Fig. 5B illustrates external dimensions of injector **500** in accordance with some embodiments of the current invention. In some embodiments, the distance **529a** from the longitudinal ccntcr of mass **549a** of the injector and the adhesive surface **578** may range for example between 50 ± 10 mm. The distance **529b** between the lateral center of mass **549b** of the injector and the center of adhesion **549c** on base **542** of the apparatus (when the weighted center of force on the adhesive when the injector is twisted off the skin for example in the direction of the arrow **583** in Fig. 5B) may range, for example between 12.5 ± 4 mm. The width **529c** of base **542** may range, for example, between 60 ± 15 mm. There may optionally be a semi-stiff membrane **545** that may form a skirt extending between 0 and 20 mm beyond the edge of base **542.** (For example the skirt may be made of plastic, for example Polyethylene terephthalate (PET) and/or Polycarbonate and/or ABS. The thickness of the skirt may range for example between 0.1 to 0.8mm.). The thickness of adhesive layer **578** may range between 0.1 and 1mm. An injector may weigh for example 50 ± 20 g. Then the resting torque adhesive when the injector is adhered to a vertical object will be approximately 50 mm × 50 g = 2500 g×mm. The strength of adhesion necessary to hold the injector to the patient will be approximately 2500 g×mm/12.5mm = 200 g. In some embodiments, movements of the user may place a considerably stress on the injector than the static stress. For example an adhesive may be provided to give a total adhesive strength ranging between 500 - 1500 g.

In some embodiments, the adhesive will be less strong and/or maybe easier to remove. For example the strength of the adhesive may be less than 500 g (for example the user may have to hold the injector with his hand to prevent it from falling, especially when the user is moving). Alternatively or additionally the adhesive may not include semi-stiff membrane **545** and/or the skirt.

In some embodiments, the adhesive may include a semi stiff skirt. The skirt may make the injector more stable. Alternatively or additionally, the adhesive may be connected to a stiff base (for example the base of the injector) without a semi-stiff skirt. For example, an embodiment without a semi stiff skirt may be easier to remove after the end of injection.

Figs. 5C and 5D illustrate an exemplary method of forming extenders **586a,b** from adhesive cover **589.** For example, adhesive cover may include three folded portions including for example an adhered portion **585,** and/or two extenders **586a,b.** For example, adhesive cover may include three holes **587a,b,c.** When folded, the extenders **586a,b** may fold over the adhered portion **585.** Holes **587b,c** in extenders **586a,b** may line up with hole **587a** in adhered portion **585.** In some embodiments, adhered portion **585** will be adhered to base **542** with the needle opening accessible through holes **587a-c.** A cap (for example safety cap **592**) may protrude through holes **587a-c.** The cap may optionally be attached to portions **586b,c.** The safety cap may pass freely through the needle opening and/or hole **587a** and/or not be attached to portion **585** of adhesive cover **589.** For example, the needle opening and/or holes may range between 1 and 3 mm wide and/or between 3 and 7 mm wide and/or between 7 and 15 mm wide.

Figs. 5E-H illustrate removal of an exemplary safety cap **592,** needle cover **591** and/or adhesive cover **589.** For example, while safety cap **592** is mounted to needle cover **591,** safety cap **592** may prevent deployment and/or activation of the injector. For example, safety cap **592** and handle **594** may supply a convenient means of removing needle cover **591** and/or adhesive cover **589.**

Fig. 5E illustrates injector **500** in a safe state for storage and/or transport. Needle cover **591,** safety cap **592** and adhesive cover **589** are in place. Adhesive cover **589** is folded for example as illustrated in Fig. 5D. Needle cover **591** (not seen in the drawing) may optionally preserve the sterility of needle **560.** Safety cap **592** may optionally surround and/or grasp needle cover **591.** Safety cap **592** may prevent inadvertent activation of the injector and/or protect users from a needle stick hazard.

Fig. 5F illustrates the beginning of removal of safety cap **592.** A user optionally pulls handle **594** away from needle **560.** Handle **594** pulls needle cover **591** out the needle hole of injector **500** and through hole **587a.** As cap **592** is pulled away from base **542,** extenders **586a,b** unfold while adhered portion **585** remains adhered to base **542.**

Fig. 5G illustrates that as safety cap **592** is pulled further away from base **542** extenders **586a,b** pull and peel opposite edges of adhered portion **585** away from base **542.** In some embodiments, pulling opposite edges of adhered portion **585** may balance the forces on safety cap **592.** The balanced forces may produce a net force on cap **592** that is substantially perpendicular to base **542.**

Fig. 5H illustrates safety cap **592** and adhesive cover **589** fully removed from injector **500,** such that injector **500** is enabled and/or ready to adhere to a patient and/or ready for activation.

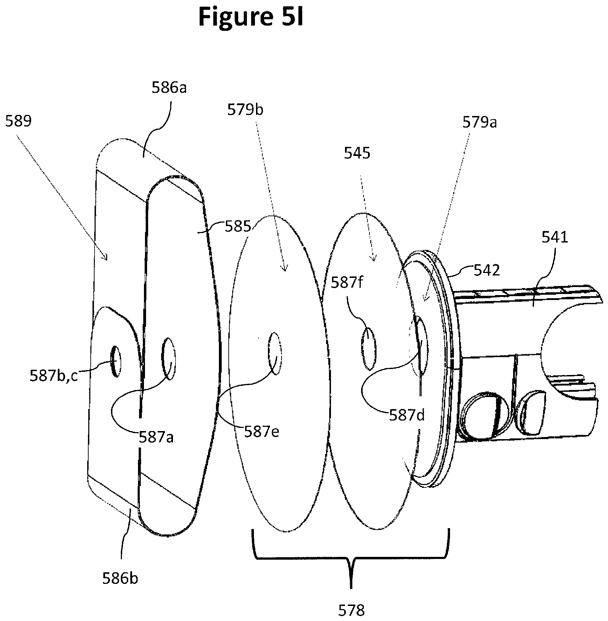

Fig. 5I illustrates details of the structure of an active zone including an adhesive **578** formed on a surface in accordance with some embodiments of the current invention. In some embodiments a wall of a device (for example base **542**) may have an active surface (for example adhesive **578**). Optionally the adhesive may be formed in layers. For example, as illustrated in Fig. 5I, adhesive **578** includes three layers. A first layer includes a dual sided adhesive **579a.** Adhesive **579a** may, for example, join a semi-stiff membrane **545** to base **542** Membrane **545** may extend beyond the edges of base **542.** A semi-stiff extension beyond the edges of base **542** may in some embodiments make the injector adhere more strongly the skin of a user. A semi-stiff extension beyond the edges of base **542** may in some embodiments make it more difficult to peel adhesive cover **589** from adhesive **578.** The angle of peeling of adhesive cover **589** and/or the stiffness of membrane **545** may be adjusted (for example the angle of peeling made shallower and/or the membrane made stiffer) to facilitate peeling adhesive cover **589** without undue bending of membrane **545.** For example, the adhesive cover removal force may range between 10-150 gr/cm. The adhesive cover removal force may be lower than the bending inertia of the adhesive membrane 545. For example this may prevent bending of membrane **545** when removing the adhesive cover. An external face of membrane **545** may optionally include an active surface, for example an adhesive **579b.** An active surface for the purpose of this application may be a surface that has active modality wherein the surface interacts with an external element facilitating the functioning of the device and an inactive modality wherein it substantially does not interact with the external element. For example, prior to enablement of injector **500** (for example see enabled **332** Fig. 3) adhesive **579b** may be inactive and/or protected by adhesive cover **589.** When the injector is active (for example, see active **333** Fig. 3) adhesive **579b** may be actively interacting with a patient. For example adhesive **579b** may adhere to the patient and/or support the injector on the patient. Alternatively or additionally, an active surface may include a drug for example an antiseptic and/or an anesthetic and/or an active surface may include a coupling agent for example a electrically conductive material, and/or an active surface may include an indicator material for example a pH sensitive dye and/or a heating material.

In some embodiments, a coupler (for example safety cap **592** see, for example, Figs. 5A and 5G) may be used to open a seal of a protected enclosure (for example needle cover **591** for example see Fig. 5A) and/or surface cover **589.** For example, after installing syringe **546** and/or needle **560** in a sterile state sealed by needle cover **591,** a proximal end safety cap **592** may be inserted through holes **587e**, **587f** and **587d** in adhesive **579b,** membrane **545** and base **542** respectively. The proximal end of safety cap **592** may, for example, snap over and/or attach to needle cover **591.** The distal end of safety cap **592** may, for example, remain protruding external to injector **500.** Optionally, extenders **586a,b** may be folded over and/or holes **587b,c** may be attached to the distal end of safety cap **592.** For example, extenders **586a,b** may be clamped to the distal end of safety cap **592** using handle **594** (see for example Figs. 5A, 5B, 5G).

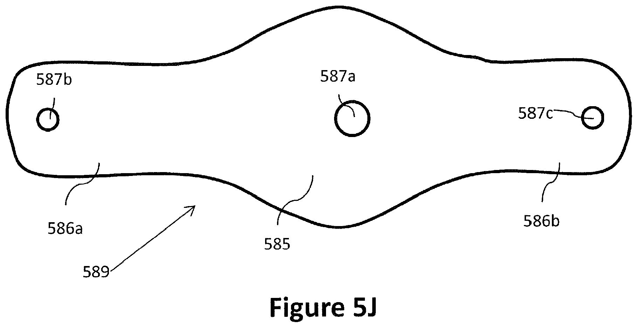

Fig. 5J illustrates adhesive cover **589** spread out flat in accordance with some embodiments of the current invention. In the embodiment of Fig. 5J, adhered portion **585** of cover **589** may optionally be wider than extenders **586a,b.** For example, adhered portion **585** may have a width ranging 70±20 mm and extenders **586a,b** may have a width ranging 30±10 mm. The length of each extender **586a,b** may range for example 180±80 mm. Attachment holes **587b,c** may have a diameter of for example 6±2 mm. Insertion hole **587a** may have a diameter of for example 10±4 mm. Alternatively or additionally, holes **587a,b,c** may be centered on the device and/or they may be off center. Cover **589** may optionally be made of and/or coated with an adhesive inert material.

Various aspects or features illustrated herein with respect to a particular embodiment may be combined with other alternative embodiments. For example, needle **560** of injector **500** is illustrated perpendicular to base **542.** Alternatively or additionally, needle **560,** may optionally be mounted at an acute angle to base **542.** Needle protectors and/or protective covers may vary in geometry. Various retraction mechanisms may used, for example spring driven mechanism and/or clip and/or a screw driven rotary mechanism.

In some embodiments a syringe (for example syringe **546**) may be preloaded with a medicine. For example the volume of preloading medicine may range between 0.5 and 1ml and/or between 1 and 5 ml and/or greater than 5 ml of medicine. Preloading may optionally be performed on standard syringe equipment and using standard filling procedures. Optionally the syringe may be a standard type syringe. For example preloading may be done in an aseptic environment. In some embodiments, a syringe medicine container and/or needle may be filled and sealed under aseptic and/or sterile conditions, for example in an aseptic room. For example the syringe may be sealed by a needle protector and/or a plunger. Optionally, the syringe, with the fluid path in a sealed and/or protected state may be taken from the aseptic filling room and installed into an injector. Optionally, the fluid path may not require sterilization after being removed from the filling room and/or after installation into the injector. In some embodiments, the fluid path of the injector may include the medicine container and/or the needle. For example, in operation, medicine stored in the container may pass directly from the container to the needle and/or from the needle directly to the patient. Optionally, the entire fluid path may be in a complete and/or sterile and/or assembled and/or protected state prior to and/or during filling of the container.

### 6 Single folded surface cover

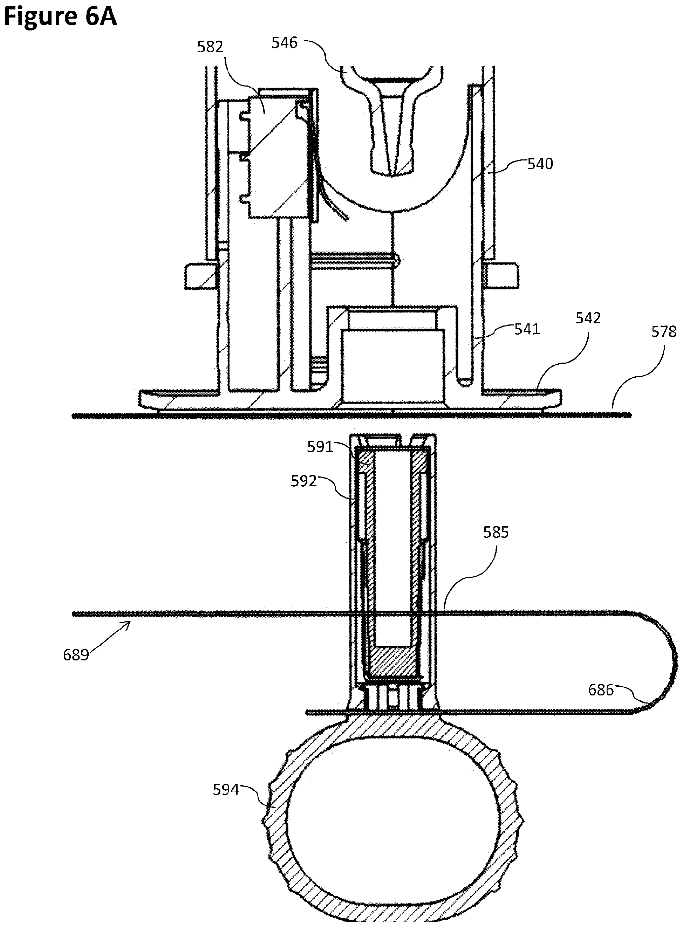

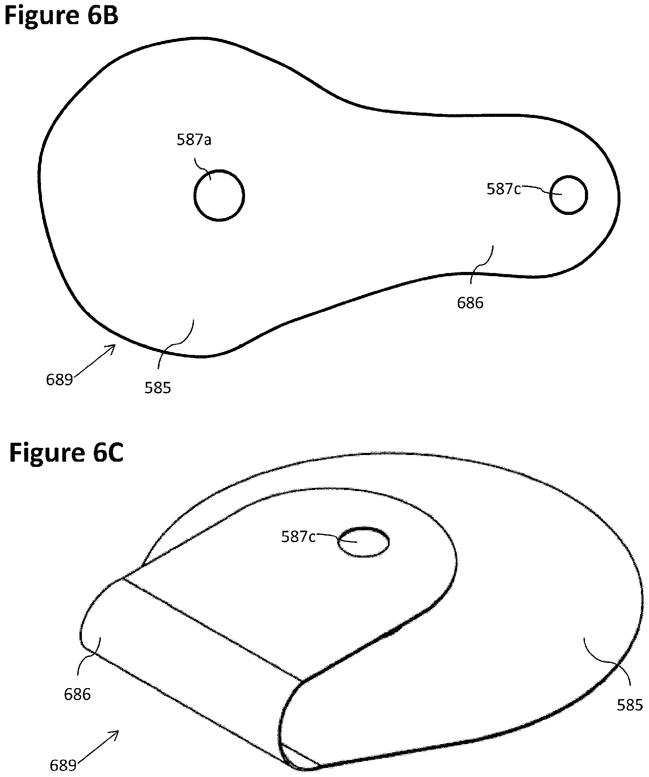

Figs. 6A-C illustrate an alternate adhesive cover **689** having a single fold and/or extender in accordance with some embodiments of the current invention. In Figs. 6A-C, parts having similar geometry and function to counterparts in Fig. 5A-K are marked with the same number as their counterparts in Figs. 5A-K. One difference between adhesive cover **689** and adhesive cover **589** is that adhesive cover **689** optionally includes a single extender **686.** Extender **686** optionally peels adhered portion **585** from one edge and/or may not balance peeling forces.

Fig. 6A illustrates adhesive cover **689** attached to safety cap **592** in accordance with some embodiments of the current invention. Fig. 5B illustrates adhesive cover **689** stretched out flat in accordance with some embodiments of the current invention. Fig. 6C illustrates a perspective view of adhesive cover **689** in a folded state in accordance with some embodiments of the current invention. Alternatively or additionally an adhesive cover may include three for or more extenders. Alternatively or additionally an adhered portion **585** may have a shape other than circular, for example oval, rectangular and/or irregular.

### 7 Alternative surface covers

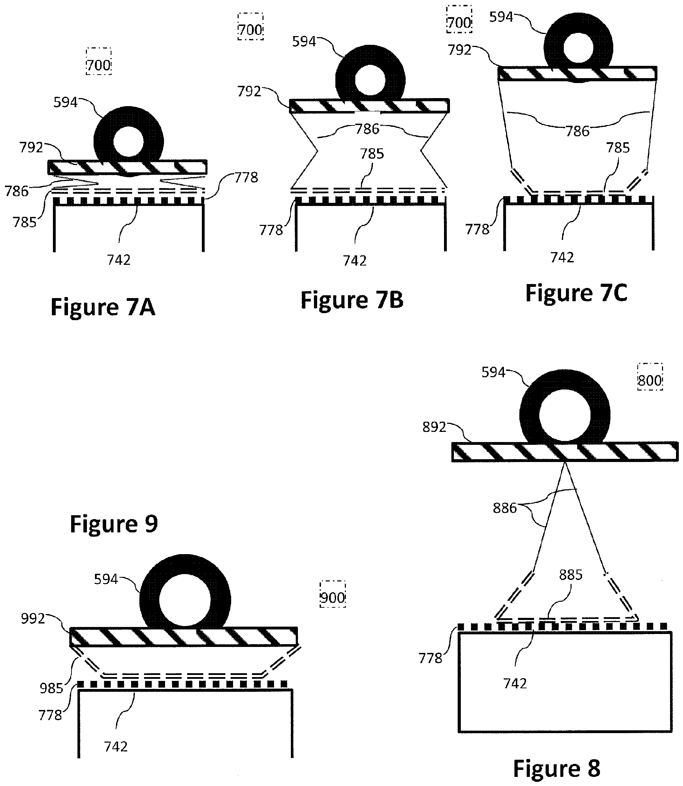

Figs. 7A-D, and Figs. 8, and 9 illustrate examples of alternate geometries for an adhesive cover peeler in accordance with some embodiments of the current invention. An adhesive peeler may include for example extenders attached to an outer edge of a safety cap and/or may not include extenders and/or may include extenders from a central point on the safety cap.

Figs. 7A-C illustrated three stages of synchronized removal of a cap and peeling an adhesive cover in accordance with some embodiments of the current invention. For example, a stiff cap **792** may cover a surface **742** of a device **700.** An active surface for example adhesive **778** may be formed on the outer surface of the device **700.** In some embodiments, active surface for example adhesive **778** may be covered a protective cover **785.** One or more extenders **786** may optionally join cap **792** to protective cover **785.** For example in device **700,** extenders **786** join the edges of cap **792** to the edges of protective cover **785.** When cap **792** is closed onto surface **742,** extenders **786** may optionally fold up (for example as illustrated in Fig. 7A). As cap **792** is pulled away from surface **742,** extenders **786** may optionally unfurl (for example as illustrated in Fig. 7B). Pulling cap **792** further from surface **742** optionally peels cover **785** from surface **742** (for example as illustrated in Fig. 7C). As cover **785** peels off of surface **742** the lower ends of extenders **786** may be pulled inward and/or approach each other.

Fig. 8 illustrates an alternate embodiment of synchronized removal of a cap and peeling an adhesive cover in accordance with some embodiments of the current invention. For example, in device **800** of Fig. 8, extenders **886** join a central point of cap **892** to the edges of surface cover **885.** As cap **892** is pulled away from surface **742,** surface cover **885** is peeled from the active surface for example adhesive **778.**

In some embodiments, extenders (for example extenders **786** and/or **886**) may be extensions of a surface cover (for example cover **785** and/or **885**) (for example similar to extenders **586** illustrated in Figs. 5A-K). For example, the extenders and the surface cover may be made of a flexible material. In some embodiments extenders may be a part of the cap. The cap and the extenders may be integrally formed of a single piece of flexible material. Alternatively or additionally, extenders may be formed separately from the cap and/or the surface cover. For example flexible extenders may be attached rigidly to the cap and/or the surface cover. Alternatively or additionally, extenders (whether the extenders are rigid and/or flexible) may be attached to the cap and/or the surface cover with a flexible attachment mechanism, for example a hinge and/or fold.

Fig. 9 illustrates an alternate embodiment of synchronized removal of a cap and peeling an adhesive cover in accordance with some embodiments of the current invention. For example, in device **900** of Fig. 9, the edges of a surface cover **985** are attached directly to a stiff cap **992** without extenders. As cap **992** is pulled away from surface **742,** surface cover **985** is peeled from the active surface for example adhesive **778.** Optionally, surface cover **985** may be elastic. The elasticity of cover **985** may optionally make it easier to distance cap **992** giving more leverage to the peeling. For example, as cover **985** is pulled away from surface **742,** surface cover may stretch. As surface **742** surface cover may stretches, the angle between the raised ends of surface cover **985** and surface **742** may increase increases the leverage of the peeling force.

### 10 Patch injector with synchronized unsealing and surface peeling

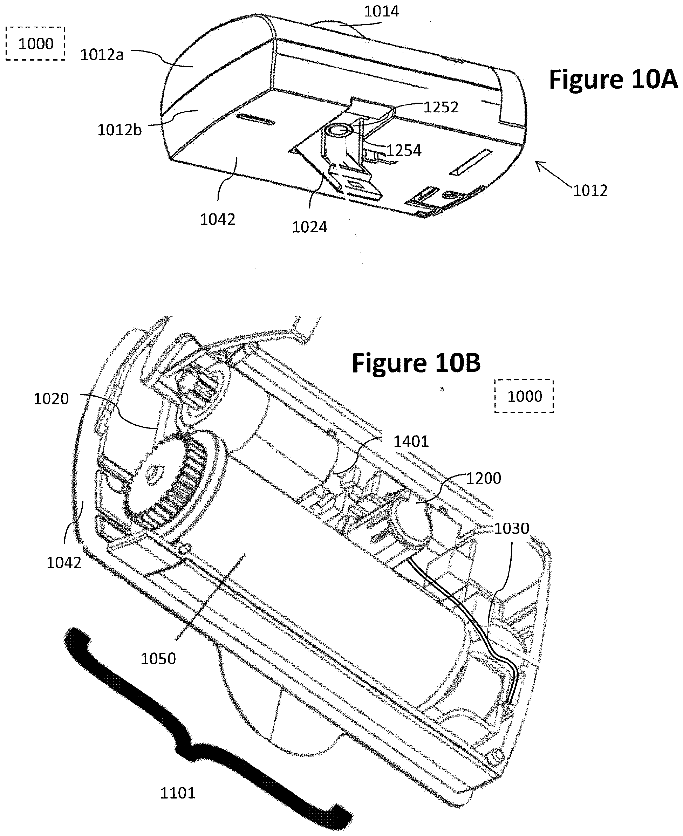

Figs. 10A-D illustrate a compound device having synchronized removal of a seal and a surface cover in accordance with some embodiments of the present invention. For example the a compound device may be a patch injector including for example a protected internal mechanism (for example an sterile injection needle sealed in an enclosure) and an active external surface (for example an adhesive surface). A surface protector covering may be joined to a seal of a needle enclosure. Peeling the surface protector may be synchronized with unsealing the protective enclosure.

An exemplary patch injector, pump **1000** is, for example, a drug delivery systems capable of dispensing a fluid to a user upon activation. Such drug delivery systems include, for example, injection systems, infusion pumps, bolus injectors, and the like. FIGS. 10A and 10B show an exemplary drug delivery device in accordance with at least one embodiment of the present invention. The drug delivery device may be utilized to administer delivery of a drug treatment into a body of a user. Drug pump **1000** may optionally include a pump housing **1012a,b.** Pump housing **1012a,b** may include one or more housing subcomponents which are fixedly engageable to facilitate easier manufacturing, assembly, and operation of the drug pump. For example, drug pump **1000** includes a pump housing **1012a,b** which includes an upper housing **1012a** and a lower housing **1012b.** Drug pump **1000** may further include an activation mechanism **1014.** As shown in FIG. 10B, a drug pump may further include assembly platform **1020,** drive mechanism **1101** having drug container **1050,** insertion mechanism **1200,** sterile fluid conduit **1030,** and power and control system **1401.** One or more of the components of such drug pumps may be modular in that they may be, for example, pre-assembled as separate components and configured into position onto the assembly platform **1020** of the drug pump **1000** during manufacturing.

Drug pump **1000** is configured such that, upon activation by a user by depression of activation mechanism **1014,** the drug pump is initiated to: insert a fluid pathway into the user; enable, connect, and/or open necessary connections between a drug container, a fluid pathway, and a sterile fluid conduit; and/or force drug fluid stored in the drug container through the fluid pathway and fluid conduit for delivery into a user. One or more optional safety mechanisms may be utilized, for example, to prevent premature activation of the drug pump. For example, an optional on-body sensor **1024** may be provided in one embodiment as a safety feature to ensure that the power and control system **1401,** and/or the activation mechanism, cannot be engaged unless the drug pump **1000** is in contact with the body of the user. In one such embodiment, the on-body sensor **1024** is located on the bottom of lower housing **1012b** (for example on a patient contact surface **1042**) where it may come in contact with the user's body. When the on-body sensor **1024** is depressed activation may optionally be permitted.

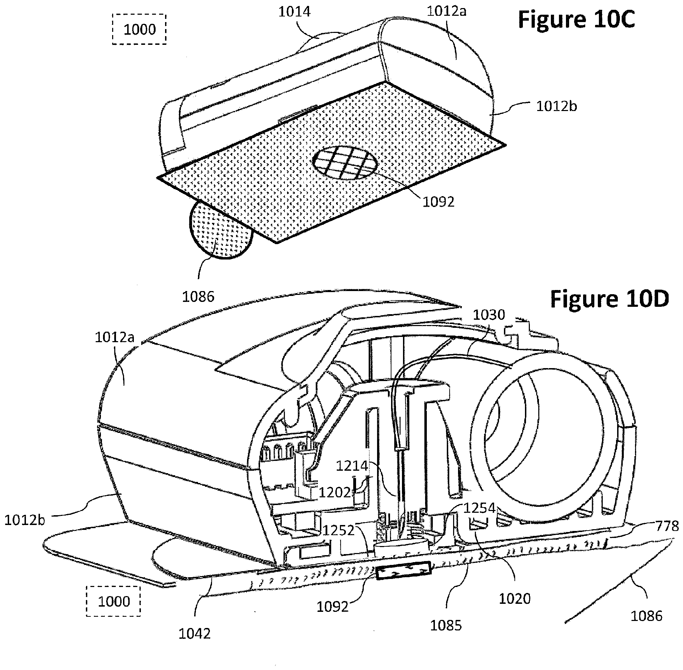

In some embodiments, insertion mechanism **1200** may include a sterile enclosure **1202** (for example as illustrated in Fig. 10D) having a base **1252.** Base **1252** may be connected to assembly platform **1020** to integrate the insertion mechanism into the drug pump **1000** (as shown for example in FIG. 10A). In some embodiments, the bottom of base **1252** may include a seal **1254** that, at least in one embodiment, is removable prior to use of the drug pump **1000.** For example at least a part of the fluid path of pump **1000** may be produced and/or sealed in enclosure **1202.** The assembled fluid path and/or sealed enclosure may be installed into housing **1012a,b** as a complete component.

In some embodiments the insertion mechanism **1200** may further include, for example, an insertion biasing member and/or a needle **1214** (see for example Fig. 10D). Needle **1214** may optionally connect to sterile fluid conduit **1030** to permit fluid flow through needle **1214** and/or a cannula, and into the body of the user during drug delivery. Optionally, there may be a sterile septum under seal **1254.** The septum may protect needle **1214** and/or keep it sterile until needle **1214** is inserted through the septum into a patient.

Fig. 10C illustrates a view of pump **1000** from the side of patient contact surface **1042.** In some embodiments, after assembling insertion mechanism **1200** into pump **1000**, an adhesive **778** layer (for example as illustrated in Fig. 10D) may be formed on and/or adhered onto an external side of patient contact surface **1042.** Adhesive **778** may optionally be formed on and/or adhered onto an external surface of seal **1254.** After forming the adhesive **778** layer, adhesive cover **1085** along with coupler **1092** may be placed over the adhesive. The adhesive may be protected from the external environment by surface cover **1085.** Surface cover **1085** may be joined to seal **1254** by a coupler (for example coupler **1092** as illustrated for example in Figs. 10C and 10D). In alternative embodiments, details of construction may differ, for example, adhesive may be formed on an external surface of drug delivery device before installation of insertion mechanism **1200.**

In accordance with some embodiments (for example in the exemplary embodiment of Fig. 10D), a user enables the injector by grasping a tab **1086** and peeling off adhesive cover **1085** with coupler **1092.** Optionally, adhesive cover **1085** is made of an adhesive inert material. When adhesive cover **1085** is peeled off, adhesive **778** remains on most of the contact surface **1042.** On the other hand, coupler **1092** may adhere via adhesive **778** and/or to seal **1254.** When cover **1085** and/or coupler **1092** are peeled off, seal **1254** may be come off with coupler **1092.** For example there may be a hole in on body sensor **1024** through which seal **1254** and coupler **1092** are connected.

### 11 Unsealing mechanism and surface protector

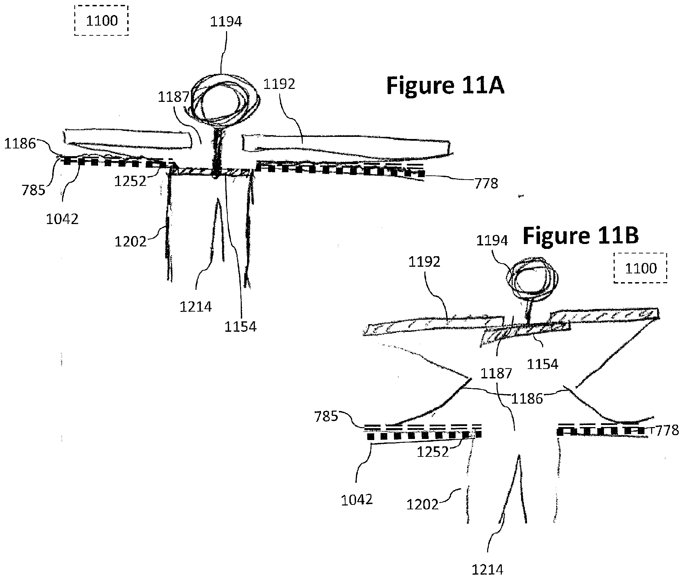

Figs. 11A,B illustrate an alternative synchronized system **1100** for peeling a surface protector and unsealing an enclosure in accordance with some embodiments of the current invention. Optionally, system **1100** is compatible and/or may be used with a patch injector such as pump **1000.** For example, after mounting needle insertion mechanism **1200,** and outside surface of the device may be formed and/or adhered to a patient contact surface **1042.** A surface cover **785** may be placed over adhesive **778** for example protecting adhesive **778** from the external environment. A hard cap **1192** may also be placed over the surface (for example as illustrated in Fig. 11A). Cap **1192** may be attached to surface cover **785** by extenders **1186.** A handle **1194** may be inserted through a hole **1187** in cap **1192** and/or cover **785** and/or adhesive **778.** Handle **1194** may be attached to seal **1154.**

In accordance with some embodiments (for example as illustrated in Figs. 11A,B) a user enables a device by pulling handle **1194** away from surface **1042.** Pulling handle may optionally pull up seal **1154.** Seal **1154** may optionally be larger than hole **1187** in cap **1192.** Pulling seal **1154** away from surface **1042** may also pull cap **1192** away. As cap **1192** is pulled away from surface **1042** extenders **1186** unfurl and/or peel surface cover **785** from surface **1042.** For example, extenders **1186** may function in a manner similar for example to extenders **786** of Fig. 7A.

### 12 Patch injector with synchronized removal of seal and adhesive cover

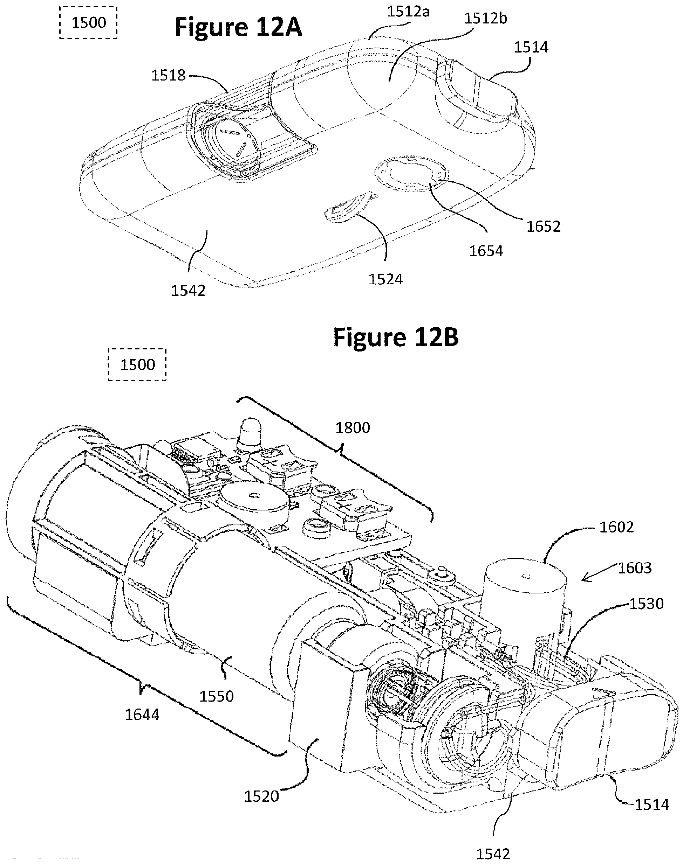

Figs. 12A-C illustrate an alternative patch injector pump **1500** having synchronized removal of a seal and a surface cover in accordance with some embodiments of the present invention.

An exemplary patch injector, pump **1500** is, for example, a drug delivery systems capable of dispensing a fluid to a user upon activation. Such drug delivery systems include, for example, injection systems, infusion pumps, bolus injectors, and the like. FIGS. 12A and 12B show an exemplary drug delivery device in accordance with at least one embodiment of the present invention. The drug delivery device may be utilized to administer delivery of a drug treatment into a body of a user. Drug pump **1500** may optionally include a pump housing **1512a,b.** Pump housing **1512a,b** may include one or more housing subcomponents which are fixedly engageable to facilitate easier manufacturing, assembly, and operation of the drug pump. For example, drug pump **1500** includes a pump housing **1512a,b** which includes an upper housing **1512a** and a lower housing **1512b.** Drug pump **1500** may further include an activation mechanism **1514,** and a window **1518.** Window **1518** may be any translucent or transmissive surface through which the operation of the drug pump may be viewed. As shown in FIG. 12B, drug pump further includes assembly platform **1520,** sterile fluid conduit **1530,** drive mechanism **1644** having drug container **1550,** insertion mechanism **1603,** fluid conduit **1530,** and power and control system **1800.** One or more of the components of such drug pumps may be modular in that they may be, for example, pre-assembled as separate components and configured into position onto the assembly platform **1520** of the drug pump **1500** during manufacturing.

Drug pump **1500** is configured such that, upon activation by a user by depression of activation mechanism **1514,** the drug pump is initiated to: insert a fluid pathway into the user; enable, connect, and/or open necessary connections between a drug container, a fluid pathway, and a sterile fluid conduit; and/or force drug fluid stored in the drug container through the fluid pathway and fluid conduit for delivery into a user. One or more optional safety mechanisms may be utilized, for example, to prevent premature activation of the drug pump. For example, an optional on-body sensor **1524** may be provided in one embodiment as a safety feature to ensure that the power and control system **1401,** and/or the activation mechanism, cannot be engaged unless the drug pump **1500** is in contact with the body of the user. In one such embodiment, the on-body sensor **1524** is located on the bottom of lower housing **1512b** (for example on a patient contact surface **1542**) where it may come in contact with the user's body. Upon displacement of the on-body sensor **1524,** depression of the activation mechanism is permitted.

In some embodiments, insertion mechanism **1603** may include a sterile enclosure **1202** having a base **1652.** Base **1652** may be connected to assembly platform **1520** to integrate the insertion mechanism into the drug pump **1500** (as shown for example in FIG. 12A). The connection of the base **1652** to the assembly platform **1520** may be, for example, such that the bottom of the base is permitted to pass-through a hole in the assembly platform to permit direct contact of the base to the body of the user. In some embodiments, the bottom of base **1652** may include a seal **1654** that, at least in one embodiment, is removable prior to use of the drug pump **1500.** For example at least a part of the fluid path of pump **1500** may be produced and/or sealed in enclosure **1602.** The assembled fluid path and/or sealed enclosure may be installed into housing **1512a,b** as a complete component.

In some embodiments the insertion mechanism **1603** may further include, for example, an insertion biasing member and/or a needle **1214.** Needle **1214** may optionally connect to sterile fluid conduit **1530** to permit fluid flow through needle **1214** and/or a cannula, and into the body of the user during drug delivery. Optionally, there may be a sterile septum under seal **1654.** The septum may protect needle **1214** and/or keep it sterile until needle **1214** is inserted through the septum into a patient.

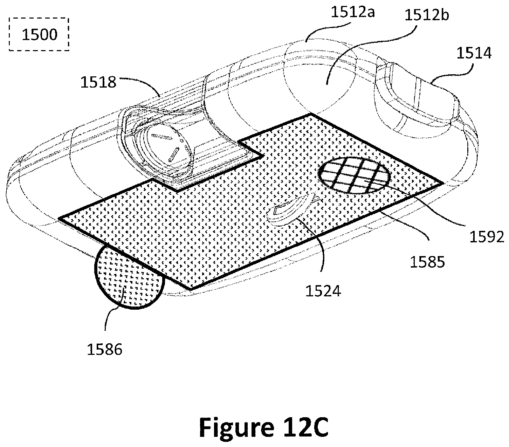

Fig. 12C illustrates a view of pump **1500** from the side of patient contact surface **1542.** In some embodiments, after assembling insertion mechanism **1603** into pump **1500,** an adhesive **778** layer (for example as illustrated in Fig. 12D) may be formed on and/or adhered onto an external side of patient contact surface **1542.** Adhesive **778** may optionally be formed on and/or adhered onto an external surface of seal **1654.** After forming the adhesive **778** layer, adhesive cover **1585** along with coupler **1592** may be placed over the adhesive. The adhesive may be protected from the external environment by surface cover **1585.** Surface cover **1585** may be joined to seal **1654** by a coupler (for example coupler **1592** as illustrated for example in Figs. 12C and 12D). In alternative embodiments, details of construction may differ, for example, adhesive may be formed on an external surface of drug delivery device before installation of insertion mechanism **1603.**

In accordance with some embodiments (for example in the exemplary embodiment of Fig. 12D), a user enables the injector by grasping a tab **1586** and peeling off adhesive cover **1585** with coupler **1592.** Optionally, adhesive cover **1585** is made of an adhesive inert material. When adhesive cover **1585** is peeled off, adhesive **778** remains on most of the contact surface **1542.** On the other hand, coupler **1592** may adhere via adhesive **778** and/or to seal **1654.** When cover **1585** and/or coupler **1592** are peeled off, seal **1654** may be come off with coupler **1592.**

### 13 Removing a Sterile needle cover

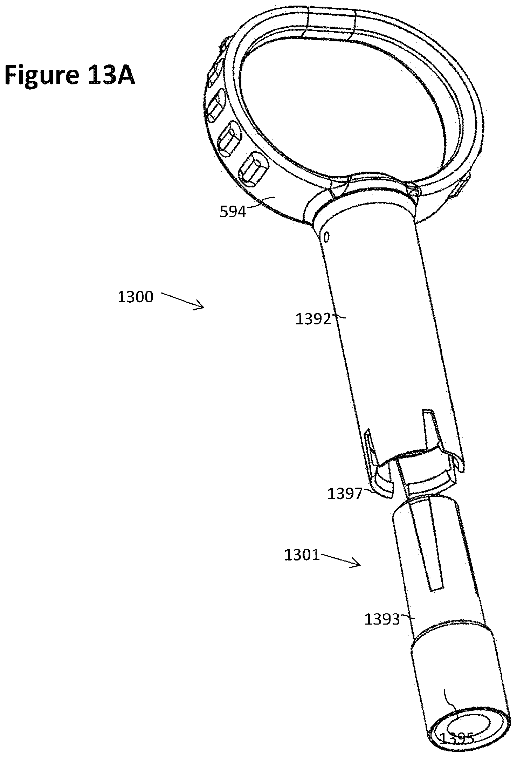

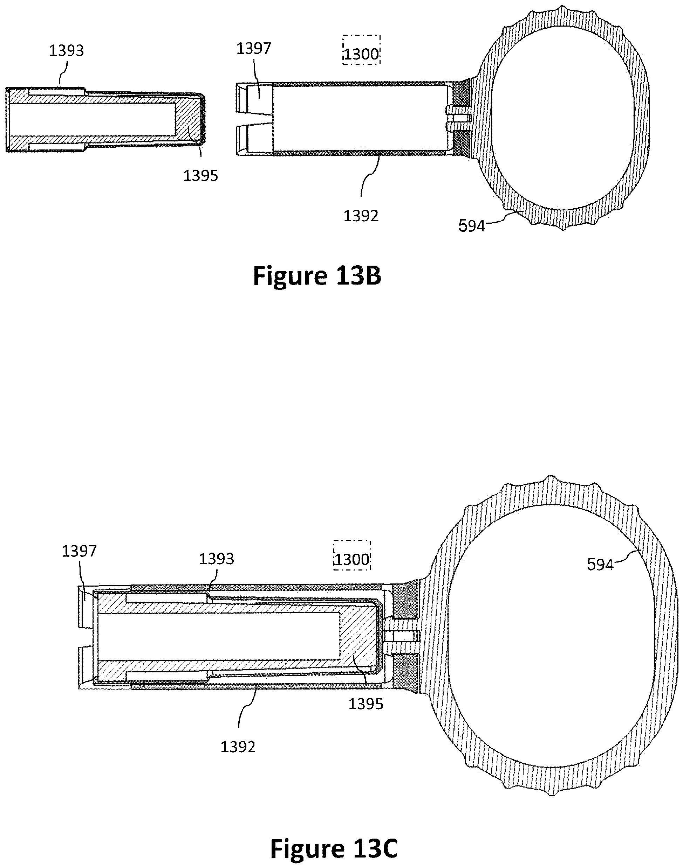

Figs. 13A-C illustrate a safety cover **1300** designed to remove a needle cover **1301** in accordance with an embodiment of the current invention. In some embodiments a needle may be installed into an autoinjector while covered with a needle cover. Subsequently, a safety cover may be installed to the injector. Removal of the safety cover may also bring about removal of the needle cover.

Fig. 13A is a perspective view of needle safety cover **1300** and associated needle cover **1301.** Optionally, needle cover **1301** includes a rigid outer shell **1393** and a sterile rubber core **1395.** When needle cover **1301** is installed over a sterilized needle, rubber core **1395** may protect the sterility of the needle and/or shell **1393** may protect the needle from causing a stick hazard. Alternatively or additionally, a needle may be covered with a single cover (either rubber or rigid) that may preserve sterility and/or prevent a stick hazard.

For example, a prefilled syringe may be installed into an autoinjector with a needle already covered with needle cover **1301.** Before shipping the injector, safety cover **1300** may be installed onto the injector. A needle protector **1392** may be pushed over shell **1393** of the needle cover. Protector **1392** may include clasps (for example clasps **1397**) which engage shell **1393.** When safety cover **1300** is removed from the injector, clasps **1397** may pull off needle cover **1301.**

In some embodiments a safety cover may include a needle protector (for safety cap **592** and/or **1392**) and/or a front cover (for example cap **1192**) and/or an adhesive cover (for example like adhesive cover **589**).

### 14 Safety cover

Figs 14A-Cµ illustrate an injector safety cover and adhesive cover in accordance with an exemplary embodiment **1400** of the present invention. The safety cover may include for example a handle **1494** and/or a needle protector **1492** and/or a front cover **1489** for covering an adhesive **1478** and/or for covering an activation zone **1442.**

Fig. 14A illustrates embodiment **1400** in a secure state for example safe for transport. The safety cover covers the activation zone **1442** and/or adhesive **1478.** Needle protector **1492** rests on a frame of housing **1440** of the injector. Putting pressure on the proximal end of the injector (which may optionally be covered by front cover **1489**) may optionally push needle protector **1492** against the frame rather than pushing activation zone **1442.** Optionally putting pressure on the proximal end of the injector will not activate the injector and/or will not unshield the needle. Needle protector **1492** may optionally act at a physical shield covering needle tip **1460.**

Fig. 14B illustrates removing the safety cover in accordance with an exemplary embodiment **1400** of the present invention. Removing the cover may optionally place the injector into an enabled state.

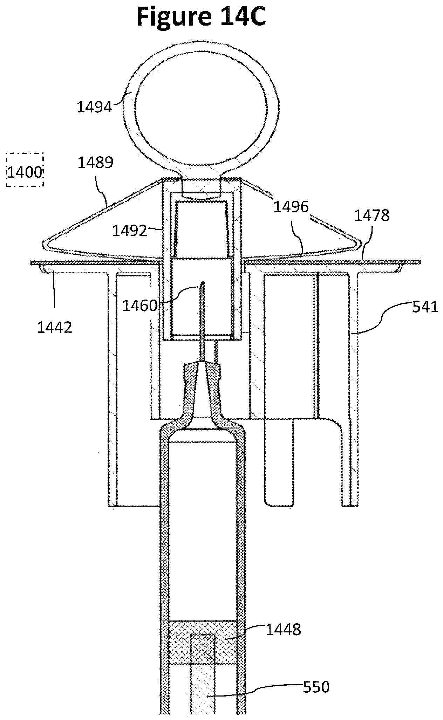

Fig. 14C illustrates peeling an adhesive protector **1496** from an adhesive **1478** in accordance with an embodiment of the present invention. In embodiment **1400** front cover **1489** serves as a peeler for an adhesive protector **1496.** For example, front cover **1489** is optionally hinged and/or flexible. Optionally, pulling handle **1494** which may optionally be located at the center of front cover **1489** does not pull an adhesive protector **1496** away from the whole surface of the adhesive **1478** all at once (an act that would require a large force to overcome the sticking force over a large surface). When handle **1494** at is pulled, the center portion of front cover **1489** optionally moves away from adhesive protector **1496.** For example cover **1489** may optionally unfold and/or flex in such a way as to peel adhesive protector **1496** bit by bit. Peeling may optionally be from an edge of adhesive **1478** and/or may optionally be towards the center.

### 15 Method of manufacture of a stabilized pen injector

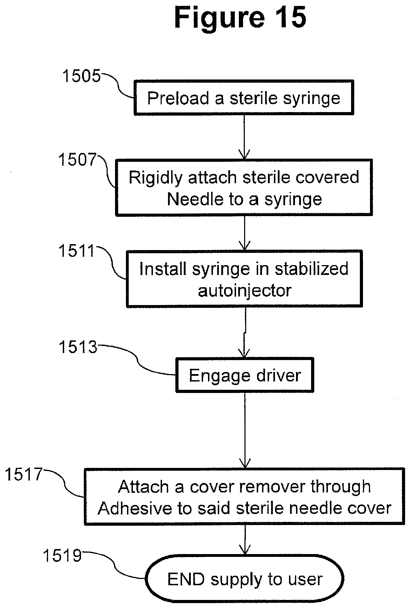

Fig. 15 is a flow chart illustration of a method of manufacture of a stabilized pen injector in accordance with an embodiment of the present invention. In the method a preloaded sterile syringe and needle may optionally be installed into an autoinjector for direct injection into a patient. For example the method of manufacture illustrated in Fig. 15 may optionally be used in manufacturing one, some and/or any of the embodiments of an injector illustrated herein above and/or below.

In some embodiments a syringe is preloaded **1505** with a medicine. For example the volume of preloading medicine may range between 0.5 and 1ml and/or between 1 and 5 ml and/or greater than 5 ml of medicine. Preloading **1505** may optionally be performed on standard syringe equipment and using standard filling procedures. Optionally the syringe may be a standard type syringe. For example preloading may be done in an aseptic environment. Optionally, a sterile needle and/or needle cover may be attached **1507** before filling the syringe. For example, the syringe may be filled while attached **1507** to a sterile needle and/or a sterile needle cover. Alternatively or additionally, the syringe may be attached **1507** to a sterile needle and/or a sterile needle cover while being filled and/or after being filled. Sterility of the needle may optionally be protected by a needle cover (for example cover **591**).

In some embodiments, the preloaded syringe with the sterile, protected needle may be installed **1511** in an autoinjector including an adhesive stabilizer (for example one of the autoinjectors described herein above). Optionally, installing **1511** the syringe may include engaging **1513** a driver to a power source, for example a battery (for example directly and/or via a motor and/or transmission) and/or a mechanical power source for example a spring.

In some embodiments, the fluid path of the injector may include the medicine container and/or the needle. For example, in operation, medicine stored in the container may pass directly from the container to the needle and/or from the needle directly to the patient. Optionally, the entire fluid path may be in a complete and/or sterile and/or assembled and/or protected state prior to and/or during filling of the container. For example, a syringe medicine container and/or needle may be filled and sealed under aseptic and/or sterile conditions, for example in an aseptic room. For example the syringe may be sealed by a needle cover and/or a plunger. Optionally, the syringe, with the fluid path in a sealed and/or protected state may be taken from the aseptic filling room and installed into an injector. Optionally, the fluid path may not require sterilization after being removed from the filling room and/or after installation into the injector.

In some embodiments, a cover remover (for example safety cover **592**) may be attached **1517** to the needle cover through a hole in the adhesive. In some embodiments, the hole in the adhesive may be surrounded by the adhesive. Alternatively or additionally, the hole may be surrounded by the adhesive on two sides and/or on three sides. For example, the adhesive may be made of two pieces, one piece on one side of the hole and another piece on another side of the hole. The autoinjector may then be ready to be supplied **1519** to a user. For example the user may use the cover remover to remove the needle cover and/or to enable the injector.

### 16 Method of stabilizing an autoinjector

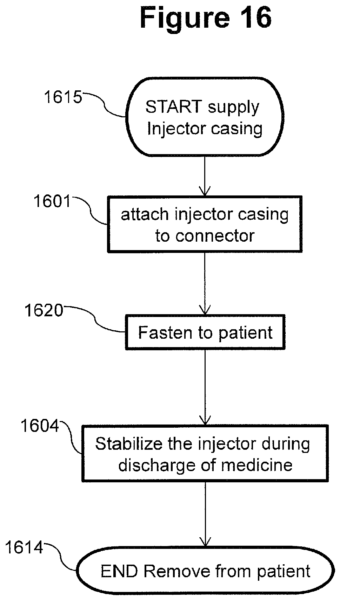

Figure 16 is a flow chart illustration of a method of adapting an injector for extended use in accordance with an embodiment of the present invention. For example an injector may be attached to a stabilizing adaptor. Stabilization may extend use of the injector to longer injection time and/or higher viscosity medicines and/or colder conditions.

In some embodiments, an injector housing may be supplied **1615.** Optionally the housing is attached **1601** to a connector. Optionally, the housing may be attached **1601** to the connector prior to assembly of autoinjector and/or prior to loading the autoinjector with medicine. For example, the autoinjector may be attached to the connector by a drug company and/or by a medical device supplier. Alternatively or additionally the injector housing may be supplied as part of an assembled autoinjector unattached to the connector. For example patient and/or a medical professional and/or a patient aid may attach **1601** the housing to the connector before use.

In some embodiments, a stabilizing adaptor may be fastened **1620** to a patient. Optionally, after attaching **1601** the injector to the adaptor, the entire assembly is fastened **1620** to the patient. For example, a patient and/or a medical professional and/or a patient aid may be supplied with a fully assembled injector and adaptor. For example the patient and/or the medical professional and/or the patient aid may fasten **1620** the assembled injector and adaptor to the patient and perform the injection. Alternatively or additionally, the adaptor may be fastened **1620** to the patient without the injector and then the injector may be attached **1601** to the adaptor while the adaptor is already fastened **1620** to the patient. For example, the adaptor may be fastened **1620** to the patient by a medical professional and/or patient aid and/or in a clinic and/or in a hospital. Later on the injector is optionally attached to the adaptor and/or activated, for example by the patient himself and/or a medical aid for example at home or elsewhere away from medical supervision.

In some embodiments, during discharge of the medicine, the autoinjector may be stabilized **1604** on the injection site of the patient by the adaptor.

In some embodiments, after injection ends, the injector and/or the adaptor may be removed **1614** from the patient. For example, the injector and the adapter may be removed **1614** together. Alternatively or additionally the injector may be disconnected from the adapter and removed from the patient while the adapter remains fastened to the patient, to be used for another injection and/or removed **1614** later.

### 17 Block diagram of an autoinjector and a stabilizing adaptor

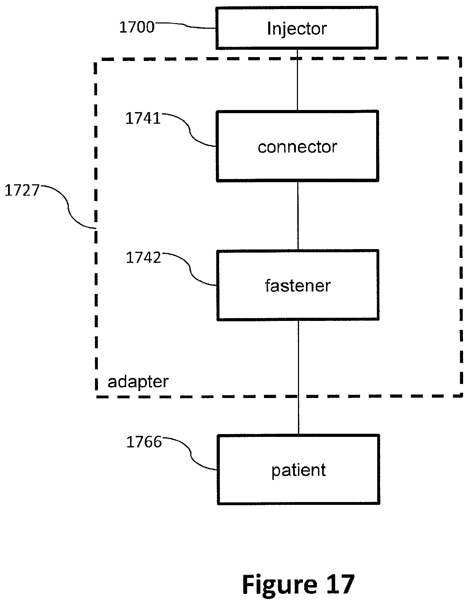

Figure 17 is a block diagram of an autoinjector **1700** and adapter **1727** in accordance with an embodiment of the present invention. Optionally an adaptor **1727** may extend operation of injector **1700.** For example, adaptor **1727** may stabilize injector **1700** on a patient allowing for longer injection times than for injector **1700** operating in an autonomous mode.

In some embodiments, adaptor **1727** may include a connector **1741** for attaching the adaptor to the injector and/or a fastener **1742** for fastening to a patient. For example, connector **1741** and fastener **1742** may be a single integrated part (for example molded out of a single piece of plastic). Alternatively or additionally, connector **1741** and fastener **1742** may be formed as two separate parts. Optionally, adapter **1727** including connector **1741** and fastener **1742** are first attached to injector **1700** and the complete assembly fastened to patient **1766** as a unit. Alternatively or additionally, adapter **1727** including connector **1741** and fastener **1742** may be fastened to the patient and injector **1700** stabilized as it is placed onto the patient. Alternatively or additionally, connector **1741** may be attached to the injector and fastener **1742** may be fastened to the patient and/or as injector **1700** is placed on the patient, connector **1741** is mated with fastener **1742** stabilizing the injector. In some embodiments there may be multiple fasteners **1742** and/or connectors **1741.**

In some embodiments, connector **1741** may attach to an outer housing of injector **1700.** For example, connector **1741** may be attached by a clamp and/or a friction fitting that grasps the walls of injector **1700.** For example the injector **1700** may have a patient contact surface on a distal end thereof and connector **1741** may be attached to the side walls of the injector **1700.** Alternatively, connector **1741** may attach to injector **1700** via an adhesive. In some embodiments, connector **1741** may be adjustable and/or adapted to attach any of a plurality to different injectors. In some embodiment, connector may be configured to attach to a specific injector. Alternatively or additionally other connection methods like magnetic force or Velcro are possible.

In some embodiments, connector **1741** may mate to fastener **1742** by a friction fitting. Alternatively or additionally connector **1741** may mate to fastener **1742** by a threaded fitting and/or a clamp and/or an interference element and/or a snap. The connection between fastener **1742** and connector **1741** may be reversible and/or may be permanent and/or irreversible (for example using a tooth and ratchet fitting). For example a ratchet and thread fittings may enable process of fixing and linkage. With a magnetic fitting linkage may occur alone. Adhesive, friction and/or Velcro® fittings may include passive actions that user should do properly. With thread, magnet, friction and/or Velcro it may be simple to disengage the device from the adaptor for example for re-use.

### 18 States of an autoinjector and extending adaptor

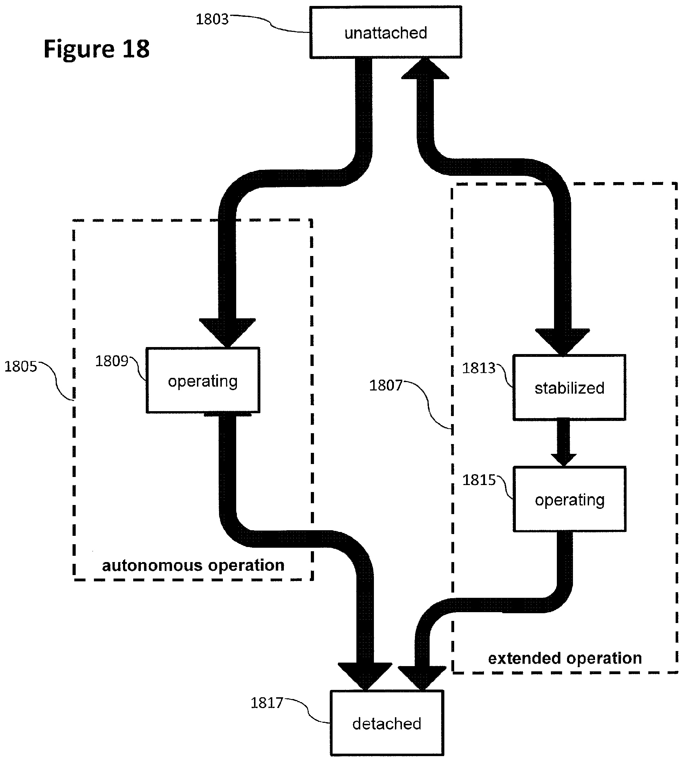

Figure 18 is a state diagram of an autoinjector and extending adaptor in accordance with an embodiment of the current invention. An autoinjector may have two or more optional operating states. For example the injector may operate in an autonomous state **1805** and/or in an operationally extended state **1807.** Operationally extended state **1807,** optionally includes operation of the injector extended to longer times and/or more viscous medicines and/or larger injection volumes. In some embodiments, operational extended state **1807** may include extension of the length of the injector. Alternatively or additionally, operational extended state **1807** may not include extension of the length of the injector. Prior to use the injector may have an unattached **1803** state and/or after use the injector may have a detached **1817** state.

In some embodiments, an injector may operate **1809** in an autonomous state **1805.** For example in the autonomous state the autoinjector may inject a medicine without an adaptor. Optionally in the autonomous state **1805** the injector may have a set of controls that are activated by a user in order. In the autonomous state **1805** the injector may inject a medicine in accordance with a prescribed protocol.

In some embodiments, the injector may operate **1815** in an extended state **1807.** In extended state **1807,** for example, an adaptor may be used to stabilize **1813** the injector on a patient. For example, the stabilized configuration may allow injection for longer periods of time and/or for a more dexterity limited patient. In the extended state **1807,** operation **1815** is optionally activated by the same set of controls and/or in accordance with the same protocol as in operation **1809** in the autonomous state **1805.** For example, for a higher viscosity medicament (for example a medicament at a lower temperature) using the same controls and protocol, the injector may require a longer time to discharge its payload. For example, extended operation **1815** may be very useful when an injector has been tested and/or approved for operation **1809** in the autonomous state **1805,** but under some conditions (for example when the medicine is cold) the injector does not meet certain requirements. For example, the injector requires more than the maximum approved time to discharge its payload. Adding the adaptor optionally makes minimal changes in the user interface and/or operation protocol of the injector, while allowing extended operation **1815.** Thus, for example with the adaptor may allow use of the injector in extended operation **1815** under extended conditions with reduced testing and/or approval while taking advantage of previous testing and/or regulatory approval of autonomous operation **1809** under more limited conditions.

It is expected that during the life of a patent maturing from this application many relevant technologies will be developed and the scope of the terms arc intended to include all such new technologies *a priori.*

As used herein the term "about" refers to ± 5%

The terms "comprises", "comprising", "includes", "including", "having" and their conjugates mean "including but not limited to".

The term "consisting of" means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims.

## Claims

1. A compound device having an interior volume and an exterior surface comprising:
a wall (542) located between the interior volume and an exterior of the compound device;
a protected component (160) surrounded by an enclosure (1202/1602), said enclosure (1202/1602) at least partially located in the interior volume;
an opening (587) in said wall (542) exposing said enclosure (1202/1602) to said exterior;
an active area (578) formed on at least a portion of the exterior surface;
a cover (1085) shielding said active area (578) from said exterior; and,
**characterized in that**,
a seal (1254) closing said enclosure (1202/1602) and isolating said protected component (160) from said wall (542) is provided;
a coupler (1092) attaching said seal (1254) to said cover (1085) is provided, wherein said coupler (1092) connects said seal (1254) to said cover (1085) via said opening (587) and wherein said coupler (1092) is adapted to synchronize removal of said cover (1085) and unsealing of said enclosure (1202/1602).

2. The compound device of claim 1, wherein said active area (578) is adjacent to said opening (587).

3. The compound device of claim 1, wherein said active area (578) includes an adhesive (579b) and said cover (1085) shielding said active area form the exterior shields (541) said adhesive (579b).

4. The compound device of claim 1, wherein said protected component (160) includes a hypodermic needle.

5. The compound device of claim 4, wherein said seal (1254) includes a needle protective sleeve (591) and wherein said sleeve (591) is removable and adapted to unseal said enclosure when the sleeve (591) is removed.

6. The compound device of claim 4, further comprising an opening (587) in said wall (542) exposing said enclosure (1202/1602) to said exterior and wherein said hypodermic needle is directed to protrude through said opening (587) in said wall (542).

7. The compound device of claim 1, wherein said coupler (1092) is adapted to convert a movement away from the exterior surface into a peeling force on said cover (1085).

8. The compound device of claim 1, wherein said coupler (1092) is connected to an edge of said cover (1085).

9. The compound device of claim 1, wherein a volume of said enclosure (1202/1602) is smaller than a volume of said interior volume.

10. The compound device according to claim 1, wherein
the active area (578) formed on at least a portion of the exterior surface includes an adhesive (579b);
said cover (1085) shielding said adhesive (579b);
a cap placed over the surface cover (1085) and being attached thereto;
and,
wherein the cover (1085) is peelable from the active outer surface, the coupler (1092) is joining the cap to the cover (1085), and wherein the cover (1085) and the cap are adapted so that when pulling the cap and a portion of the coupler (1092) joined thereto away from the surface pulling force on the coupler (1092) is converted to a peeling force on the cover (1085) of the surface.

11. The compound device of claim 10, wherein the coupler (1092) extends through the surface cover (1085) and the cap and into engagement with the seal (1254), the cap defining an aperture and the seal (1254) being greater in size than said aperture.

12. The compound device of claim 11, wherein the surface cover (1085) defines an aperture greater in size than the cap aperture and greater in size than the seal (1254), the surface cover (1085) aperture overlapping with the cap aperture.

13. The compound device of claim 11, wherein the coupler (1092) comprises a handle (594) and an extension extending therefrom, through the surface cover (1085) and the cap and into engagement with the seal (1254), the cap and the surface cover (1085) being sandwiched between the handle (594) and the seal (1254), the cap being proximate the handle (594) and the surface cover (1085) being proximate the seal (1254).

14. The compound device of claim 10, wherein the cap comprises at least one member extending therefrom and attached to the surface cover (1085), and withdrawal of said cap away from said adhesive layer (578) unfurls said at least on member and peels the surface cover (1085) from the adhesive layer (578).

15. A method of preparing the device of claim 1 for use, the device including an active outer surface (578) and a protected component (160) isolated from a wall (542) of the device by a sealed enclosure (1202/1602); the active outer surface (578) protected by a surface cover (1085), and a coupler (1092) attaching the surface cover (1085) and the sealed enclosure (1202/1602), the method comprising:
withdrawing said coupler (1092) away from said active outer surface, and, in turn
activating the active outer surface by removing the surface cover (1085) and exposing the protected component (160) to the wall (542) of the device by unsealing the sealed enclosure (1202/1602) and synchronizing the activating and the unsealing using said coupler (1092).

16. The method of claim 15, wherein said activating includes peeling the surface cover (1085) from the active outer surface (578).

17. The method of claim 16, further comprising:
pulling said coupler (1092) away from the surface cover (1085) to form a space prior to said peeling.

18. The method of claim 16, wherein the peeling force is applied on an edge of said cover (1085).

19. The method of claim 15, wherein said unsealing includes pulling a needle protective sleeve (591) along an axis of a needle.

## Patentansprüche

1. Kombinierte Vorrichtung mit einem Innenvolumen und einer Außenfläche, umfassend:
eine Wand (542), die sich zwischen dem Innenvolumen und einer Außenseite der kombinierten Vorrichtung befindet;
ein geschütztes Element (160), das von einer Einschließung (1202/1602) umgeben ist, wobei sich die Einschließung (1202/1602) zumindest teilweise im Innenvolumen befindet;
eine Öffnung (587) in der Wand (542), die die Einschließung (1202/1602) zur Außenseite exponiert;
einen aktiven Bereich (578), der auf mindestens einem Teil der Außenfläche gebildet ist;
eine Abdeckung (1085), die den aktiven Bereich (578) gegenüber der Außenseite abschirmt;
**dadurch gekennzeichnet,**
**dass** eine Dichtung (1254), der die Einschließung (1202/1602) verschließt und das geschützte Element (160) von der Wand (542) isoliert, vorgesehen ist;
**dass** ein Verbinder (1092), der die Dichtung (1254) an der Abdeckung (1085) befestigt, vorgesehen ist, wobei der Verbinder (1092) die Dichtung (1254) über die Öffnung (587) mit der Abdeckung (1085) verbindet und wobei der Verbinder (1092) ausgebildet ist zum Synchronisieren des Entfernens der Abdeckung (1085) und des Entsiegelns der Einschließung (1202/1602).

2. Kombinierte Vorrichtung nach Anspruch 1, wobei der aktive Bereich (578) an die Öffnung (587) angrenzt.

3. Kombinierte Vorrichtung nach Anspruch 1, wobei der aktive Bereich (578) einen Klebstoff (579b) aufweist und die Abdeckung (1085), die den aktiven Bereich gegenüber der Außenseite abschirmt, den äußeren Schutz (541) des Klebstoffs (579b) bildet.

4. Kombinierte Vorrichtung nach Anspruch 1, wobei das geschützte Element (160) eine Injektionsnadel umfasst.

5. Kombinierte Vorrichtung nach Anspruch 4, wobei die Dichtung (1254) eine Nadelschutzhülse (591) umfasst und wobei die Hülse (591) abnehmbar ist und geeignet ist zum Entsiegeln der Einschließung, wenn die Hülse (591) entfernt wird.

6. Kombinierte Vorrichtung nach Anspruch 4, ferner umfassend eine Öffnung (587) in der Wand (542), die die Einschließung (1202/1602) nach außen exponiert, und wobei die Injektionsnadel derart gerichtet ist, dass sie durch die Öffnung (587) in der Wand (542) vorspringt.

7. Kombinierte Vorrichtung nach Anspruch 1, wobei der Verbinder (1092) ausgebildet ist zum Umwandeln einer von der Außenfläche wegführenden Bewegung in eine auf die Abdeckung (1085) wirkende Abziehkraft.

8. Kombinierte Vorrichtung nach Anspruch 1, wobei der Verbinder (1092) mit einem Rand der Abdeckung (1085) verbunden ist.

9. Kombinierte Vorrichtung nach Anspruch 1, wobei ein Volumen der Einschließung (1202/1602) kleiner ist als ein Volumen des Innenvolumens.

10. Kombinierte Vorrichtung nach Anspruch 1,
wobei der aktive Bereich (578), der zumindest auf einem Teil der Außenfläche gebildet ist, einen Klebestoff (579b) aufweist;
wobei die Abdeckung (1085) den Klebstoff (579b) schüzt;
wobei eine Kappe über der Oberflächenabdeckung (1085) angebracht und an dieser befestigt ist;
wobei sich die Abdeckung (1085) von der aktiven Außenfläche ablösen lässt, wobei der Verbinder (1092) die Kappe mit der Abdeckung (1085) verbindet und wobei die Abdeckung (1085) und die Kappe derart ausgebildet sind, dass beim Abziehen der Kappe und eines damit verbundenen Teils des Verbinders von der Oberfläche eine auf den Verbinder (1092) wirkende Kraft in eine auf die Abdeckung (1085) der Oberfläche wirkende Abziehkraft umgewandelt wird.

11. Kombinierte Vorrichtung nach Anspruch 10, wobei sich der Verbinder (1092) durch die Oberflächenabdeckung (1085) und die Kappe hindurch in Eingriff mit der Dichtung (1254) erstreckt, wobei die Kappe eine Öffnung definiert und die Dichtung (1254) größer als die Öffnung ist.

12. Kombinierte Vorrichtung nach Anspruch 11, wobei die Oberflächenabdeckung (1085) eine Öffnung definiert, die größer ist als die Kappenöffnung und größer als die Dichtung (1254), wobei sich die Oberflächenabdeckung (1085) mit der Kappenöffnung überlappt.

13. Kombinierte Vorrichtung nach Anspruch 11, wobei der Verbinder (1092) einen Griff (594) und eine Verlängerung aufweist, die sich von dem Griff durch die Oberflächenabdeckung (1085) und die Kappe in Eingriff mit der Dichtung (1254) erstreckt, wobei die Kappe und die Oberflächenabdeckung (1085) zwischen den Griff (594) und die Dichtung (1254) geschaltet sind und wobei die Kappe in der Nähe des Griffs (594) und die Oberflächenabdeckung (1085) in der Nähe der Dichtung (1254) liegt.

14. Kombinierte Vorrichtung nach Anspruch 10, wobei die Kappe mindestens ein Element umfasst, das sich von der Kappe erstreckt und an der Oberflächenabdeckung (1085) befestigt ist und wobei das Entfernen der Kappe von der Klebeschicht (578) das mindestens eine Element entfaltet und die Oberflächenabdeckung (1085) von der Klebeschicht (578) abzieht.

15. Verfahren zur Vorbereitung der Vorrichtung nach Anspruch 1 für die Verwendung, wobei die Vorrichtung eine aktive Außenfläche (578) und ein geschütztes Element (160) umfasst, das von einer Wand (542) der Vorrichtung durch eine abgedichtete Einschließung (1202/1602) isoliert ist; wobei die aktive Außenfläche (578) durch eine Oberflächenabdeckung (1085) geschützt ist und ein Verbinder (1092) die Oberflächenabdeckung (1085) und die abgedichtete Einschließung (1202/1602) befestigt, wobei das Verfahren umfasst:
das Zurückziehen des Verbinders (1092) weg von der aktiven Außenfläche und im Gegenzug das Aktivieren der aktiven Außenfläche durch das Entfernen der Oberflächenabdeckung (1085) und das Exponieren des geschützten Elements (160) gegenüber Wand (542) der Vorrichtung durch das Entsiegeln der abgedichteten Einschließung (1202/1602) und Synchronisieren des Aktivierens und Entsiegelns unter Verwendung des Verbinders (1092).

16. Verfahren nach Anspruch 15, wobei die Aktivierung das Abziehen der Oberflächenabdeckung (1085) von der aktiven Außenfläche umfasst.

17. Verfahren nach Anspruch 16, ferner umfassend:
das Wegziehen des Verbinders (1092) von der Oberflächenabdeckung (1085), um vor dem Abziehen einen Raum zu bilden.

18. Verfahren nach Anspruch 16, wobei die Abziehkraft auf einen Rand der Abdeckung (1085) angewendet wird.

19. Verfahren nach Anspruch 15, wobei das Entsiegeln umfasst, dass eine Nadelschutzhülse (591) entlang einer Achse einer Nadel gezogen wird.

## Revendications

1. Dispositif composé ayant un volume intérieur et une surface extérieure comprenant:
une paroi (542) située entre le volume intérieur et un extérieur du dispositif composé;
un composant protégé (160) entouré d'une enceinte (1202/1602), ladite enceinte (1202/1602) étant au moins partiellement située dans le volume intérieur;
une ouverture (587) dans ladite paroi (542) exposant ladite enceinte (1202/1602) audit extérieur;
une zone active (578) formée sur au moins une partie de la surface extérieure;
un couvercle (1085) protégeant ladite zone active (578) contre ledit extérieur; et
**caractérisé en ce que**
un joint (1254) fermant ladite enceinte (1202/1602) et isolant ledit composant protégé (160) de ladite paroi (542) est prévu ;
un coupleur (1092) fixant ledit joint (1254) audit couvercle (1085) est prévu, dans lequel ledit coupleur (1092) relie ledit joint (1254) audit couvercle (1085) via ladite ouverture (587) et dans lequel ledit coupleur (1092) est adapté pour synchroniser l'enlèvement dudit couvercle (1085) et le descellement de ladite enceinte (1202/1602).

2. Dispositif composé selon la revendication 1, dans lequel ladite zone active (578) est adjacente à ladite ouverture (587).

3. Dispositif composé selon la revendication 1, dans lequel ladite zone active (578) comprend un adhésif (579b) et ledit couvercle (1085) protégeant ladite zone active des écrans extérieurs (541) dudit adhésif (579b).

4. Dispositif composé selon la revendication 1, dans lequel ledit composant protégé (160) comprend une aiguille hypodermique.

5. Dispositif composé selon la revendication 4, dans lequel ledit joint (1254) comprend un manchon protecteur d'aiguille (591) et dans lequel ledit manchon (591) est amovible et adapté pour desceller ladite enceinte lorsque le manchon (591) est enlevé.

6. Dispositif composé selon la revendication 4, comprenant en outre une ouverture (587) dans ladite paroi (542) exposant ladite enceinte (1202/1602) audit extérieur et dans lequel ladite aiguille hypodermique est dirigée pour faire saillie à travers ladite ouverture (587) dans ladite paroi (542).

7. Dispositif composé selon la revendication 1, dans lequel ledit coupleur (1092) est adapté pour convertir un mouvement s'éloignant de la surface extérieure en une force de pelage sur ledit couvercle (1085).

8. Dispositif composé selon la revendication 1, dans lequel ledit coupleur (1092) est relié à un bord dudit couvercle (1085).

9. Dispositif composé selon la revendication 1, dans lequel un volume de ladite enceinte (1202/1602) est inférieur à un volume dudit volume intérieur.

10. Dispositif composé selon la revendication 1, dans lequel
la zone active (578) est formée sur au moins une partie de la surface extérieure comprend un adhésif (579b) ;
ledit couvercle (1085) protège ledit adhésif (579b) ;
un capuchon est placé sur le couvercle de surface (1085) et y fixé ;
et
dans lequel le couvercle (1085) est pelable de la surface extérieure active, le coupleur (1092) relie le capuchon au couvercle (1085), et dans lequel le couvercle (1085) et le capuchon sont adaptés de sorte que lors du tirage du couvercle et d'une partie du coupleur (1092) connecté à celui-ci loin de la surface la force de traction sur le coupleur (1092) est convertie en une force de pelage sur le couvercle (1085) de la surface.

11. Dispositif composé selon la revendication 10, dans lequel le coupleur (1092) s'étend à travers le couvercle de surface (1085) et le capuchon et en prise avec le joint (1254), le capuchon définissant une ouverture et le joint (1254) ayant une taille supérieure à ladite ouverture.

12. Dispositif composé selon la revendication 11, dans lequel le couvercle de surface (1085) définit une ouverture de taille supérieure à l'ouverture du capuchon et de taille supérieure au joint (1254), le couvercle de surface (1085) chevauchant avec l'ouverture du capuchon.

13. Dispositif composé selon la revendication 11, dans lequel le coupleur (1092) comprend une poignée (594) et une extension s'étendant à partir de celle-ci, à travers le couvercle de surface (1085) et le couvercle et en prise avec le joint (1254), le capuchon et le couvercle de surface (1085) étant intercalés entre la poignée (594) et le joint (1254), le capuchon étant proche de la poignée (594) et le couvercle de surface (1085) étant proche du joint (1254).

14. Dispositif composé selon la revendication 10, dans lequel le capuchon comprend au moins un élément s'étendant à partir de celui-ci et fixé au couvercle de surface (1085), et le retrait dudit capuchon de ladite couche adhésive (578) déroule ledit au moins un élément et détache le couvercle de surface (1085) de la couche adhésive (578).

15. Procédé de préparation du dispositif selon la revendication 1 pour utilisation, le dispositif comprenant une surface extérieure active (578) et un composant protégé (160) isolé d'une paroi (542) du dispositif par une enceinte étanche (1202/1602); la surface extérieure active (578) protégée par un couvercle de surface (1085), et un coupleur (1092) fixant le couvercle de surface (1085) et l'enceinte étanche (1202/1602), le procédé comprenant :
retirer ledit coupleur (1092) de ladite surface extérieure active et, à son tour, l'activation de la surface extérieure active en enlevant le couvercle de surface (1085) et en exposant le composant protégé (160) à la paroi (542) du dispositif en descellant l'enceinte scellée (1202/1602) et en synchronisant l'activation et le descellement en utilisant ledit coupleur (1092).

16. Procédé selon la revendication 15, dans lequel ladite activation comprend le pelage du couvercle de surface (1085) de la surface extérieure active (578).

17. Procédé selon la revendication 16, comprenant en outre:
tirer ledit coupleur (1092) loin du couvercle de surface (1085) pour former un espace avant ledit pelage.

18. Procédé selon la revendication 16, dans lequel la force de pelage est appliquée à un bord dudit couvercle (1085).

19. Procédé selon la revendication 15, dans lequel ledit descellement comprend tirer un manchon protecteur d'aiguille (591) le long d'un axe d'une aiguille.
